(19)

Europäisches
Patentamt
European
Patent Office
Office européen
des brevets

(11) **EP 4 083 628 B1**

(12) **EUROPEAN PATENT SPECIFICATION**

(45) Date of publication and mention
of the grant of the patent:
**11.03.2026  Bulletin 2026/11**

(21) Application number: **20908039.9**

(22) Date of filing: **25.12.2020**

(51) International Patent Classification (IPC):
*G01N 33/68* (2006.01)      *G01N 33/70* (2006.01)
*A61B 5/20* (2006.01)      *G06F 17/10* (2006.01)
*G16B 40/00* (2019.01)      *G16H 50/20* (2018.01)
*G16H 50/50* (2018.01)

(52) Cooperative Patent Classification (CPC):
**G01N 33/6812; A61B 5/201; G01N 33/70;
G16H 10/40; G16H 20/10; G16H 20/60;
G16H 20/70; G16H 50/50;** G01N 2800/347;
G06F 17/18

(86) International application number:
**PCT/JP2020/048977**

(87) International publication number:
**WO 2021/132658 (01.07.2021 Gazette 2021/26)**

(54) **METHOD AND SYSTEM FOR ESTIMATING RENAL FUNCTION**

VERFAHREN UND SYSTEM ZUM SCHÄTZEN DER NIERENFUNKTION

PROCÉDÉ ET SYSTÈME D'ESTIMATION DE LA FONCTION RÉNALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2019  JP 2019239749**

(43) Date of publication of application:
**02.11.2022  Bulletin 2022/44**

(73) Proprietors:
• **Kagami Inc.**
**Ibaraki-shi**
**Osaka 567-0085 (JP)**
• **National Institutes of Biomedical Innovation,
Health and Nutrition**
**Ibaraki-shi, Osaka 567-0085 (JP)**

(72) Inventors:
• **MITA, Masashi**
**Ibaraki-shi, Osaka 567-0085 (JP)**
• **IKEDA, Tatsuhiko**
**Ibaraki-shi, Osaka 567-0085 (JP)**
• **KIMURA, Tomonori**
**Ibaraki-shi, Osaka 567-0085 (JP)**

(74) Representative: **Engelhard, Markus**
**Boehmert & Boehmert
Anwaltspartnerschaft mbB
Pettenkoferstrasse 22
80336 München (DE)**

(56) References cited:
EP-A1- 3 081 939          WO-A1-2014/129490
WO-A1-2016/025429          WO-A1-2019/235591
JP-A- 2015 132 598          JP-A- 2016 530 532
JP-A- 2017 003 589          JP-A- 2017 207 489
JP-A- 2019 215 338          KR-B1- 101 361 038
US-A1- 2018 252 730          US-A1- 2019 317 106

• **HESAKA ATSUSHI ET AL: "D-Serine reflects
kidney function and diseases", vol. 9, no. 1, 1
December 2019 (2019-12-01), XP055935273,
Retrieved from the Internet <URL:https://www.
nature.com/articles/s41598-019-41608-0.pdf>
DOI: 10.1038/s41598-019-41608-0**

EP 4 083 628 B1

**(Cont. next page)**

- HIDEHIRO IWAKAWA ET AL: "Urinary D-serine level as a predictive biomarker for deterioration of renal function in patients with atherosclerotic risk factors", BIOMARKERS, TAYLOR AND FRANCIS, LONDON, GB, vol. 24, no. 2, 23 October 2018 (2018-10-23), pages 159 - 165, XP009530998, ISSN: 1354-750X, DOI: 10.1080/1354750X.2018.1528632
- TOMONORI KIMURA ET AL: "Chiral amino acid metabolomics for novel biomarker screening in the prognosis of chronic kidney disease", SCIENTIFIC REPORTS, vol. 6, no. 1, 18 May 2016 (2016-05-18), XP055371897, DOI: 10.1038/srep26137
- TOMONORI KIMURA, KENJI HAMASE, YURIKA MIYOSHI, RYOHEI YAMAMOTO, KEIKO YASUDA, MASASHI MITA, HIROMI RAKUGI, TERUMASA HAYASHI, YOSHI: "Chiral amino acid metabolomics for novel biomarker screening in the prognosis of chronic kidney disease", SCIENTIFIC REPORTS, vol. 6, no. 1, 1 September 2016 (2016-09-01), XP055371897, DOI: 10.1038/srep26137

**Description**

FIELD

The present invention relates to a method for estimating kidney function of a subject being evaluated, and to a system for estimating kidney function of a subject being evaluated.

BACKGROUND

Glomerular filtration rate (GFR) is a typical marker for indication of kidney function. The glomerular filtration rate represents the liquid volume filtered per minute from blood by the glomeruli, with inulin clearance considered to be the international gold standard. However, measurement of inulin clearance requires continuous drip infusion of inulin over a period of 2 hours as well as urine and blood collection multiple times, which creates a burden for both the patient and the practitioner. For routine practice in the clinic, therefore, measurement of inulin clearance is only carried out for limited situations such as donors for living kidney transplant, while for most cases it is substituted by measurement of other markers such as creatinine. Most marker values, however, diverge significantly from the actual glomerular filtration rate according to the gold standard of inulin clearance, thus interfering with accurate diagnosis of kidney disease.

Creatinine is routinely measured in the clinic as a marker for kidney function. Creatinine is the final metabolite of creatine which is necessary for muscle contraction. Creatine formed in the liver is taken up into muscle cells and partially metabolized to creatinine, transported to the kidneys through the blood, filtered by the glomeruli, and then excreted into urine in the kidney tubules without being reabsorbed. It is utilized for evaluation of kidney function because it can serve as an advantageous marker for uremia, since reduced glomerular filtration capacity leads to impaired excretion and accumulation in the blood causing its numerical value to increase. However, the amount of creatinine in blood does not appear as a clearly abnormal value until GFR has reduced by 50% or greater, and it therefore cannot be considered to be a sensitive marker.

Cystatin C is a protein of 13.36 kDa molecular weight that is produced in a fixed proportion by systemic nucleated cells, and is completely filtered out by the glomeruli and subsequently catabolized in the kidneys via reabsorption in the kidney tubules, and since it is therefore thought to be removed from the blood depending on the filtration rate, its amount in blood serves as a GFR marker. When kidney function is greatly reduced, however, the amount of increase in blood cystatin C reaches a plateau, and in end-stage kidney disease it becomes difficult to accurately evaluate kidney function.

Thus, no biomarker has yet existed that can adequately meet clinical demands for accurately measuring glomerular filtration rate for individual patients in a wide range from early to end stage by blood collection alone, without a large burden on subjects or patients.

Conventionally, D-amino acids had been considered to be absent from a mammalian body but have since been shown to be present in various tissues and to play physiological functions. It has been shown that the amounts of D-serine, D-alanine, D-proline, D-glutamic acid and D-aspartic acid in blood can serve as kidney failure markers since they vary in kidney failure patients and correlate with creatinine (NPL 1, NPL 2, NPL 3, NPL 4). It has also been disclosed that amino acids selected from the group consisting of D-serine, D-threonine, D-alanine, D-asparagine, D-allothreonine, D-glutamine, D-proline and D-phenylalanine serve as pathology index values for kidney disease (PTL 1). The aforementioned publications merely disclose that the fluctuation of D-amino acids in the blood of patients suffering from kidney disease compared to healthy persons can be used as markers for diagnosis of kidney disease, or that D-serine blood levels of subjects correlate with creatinine levels or with estimated corrected creatinine levels, but nowhere suggest that D-amino acid blood levels directly correlate with the gold standard of inulin clearance and allow estimation of glomerular filtration rate. Incidentally, while urine L-FABP, blood NGAL and urine KIM-1 have been disclosed as kidney disease markers in recent years, these do not correlate with glomerular filtration capacity.

[CITATION LIST]

[PATENT LITERATURE]

[PTL 1] International Patent Publication No. WO2013/140785 [PTL 2] US2019/317106AI

[NON PATENT LITERATURE]

[NPL 1] Fukushima, T. et al., Biol. Pharm. Bull. 18:1130(1995)
[NPL 2] Nagata Y. Viva Origino Vol.18(No.2) (1990), 15th Lecture Meeting Abstracts

[NPL 3] Ishida et al., Kitasato Medical Journal 23:51-62(1993)
[NPL 4] Yong Huang et al., Biol. Pharm. Bull. 21:(2)156-162(1998)

SUMMARY

[TECHNICAL PROBLEM]

There is a demand for a method for accurately estimating kidney function in subjects across a wider range than with conventionally known kidney function markers such as blood creatinine.

[SOLUTION TO PROBLEM]

The present inventors, focusing on D- and L-amino acids in blood and analyzing their correlation with GFR (inulin clearance), found surprisingly that D- and L-amino acid levels in blood samples from healthy persons and kidney disease patients in the early to end stages are more highly correlated with GFR (inulin clearance) than creatinine or cystatin C levels throughout all stages, and have completed this invention based on this finding.

The present invention relates to the following:

[1] A method for estimating kidney function of a subject being evaluated, wherein the method includes:
a step of estimating the kidney function of the subject being evaluated based on the value of Y calculated from the levels of D- and L-amino acids in a biological sample from the subject being evaluated, using the following formula (I):

$$Y = a_1 \cdot X_1 + a_2 \cdot X_2 + \ldots + a_n \cdot X_n + b \quad (I)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (I) is predetermined by the regression analysis using D- and L-amino acid levels in a biological samples of arbitrary subjects as the explanatory variables and glomerular filtration rates of the arbitrary subjects as the response variables.

[2] The method according to [1] above, wherein the step of estimating the kidney function of the subject being evaluated is a step of estimating the kidney function of the subject being evaluated by estimating the glomerular filtration rate of the subject being evaluated based on the Y value.

[3] The method according to [1] or [2] above, wherein the biological sample is blood, plasma or serum.

[4] The method according to any one of [1] to [3] above, wherein formula (I) with a correlation coefficient of $R \geq 0.5$, is used.

[5] The method according to any one of [1] to [3] above, wherein formula (I) with a correlation coefficient of $R \geq 0.8$, is used.

[6] The method according to any one of [1] to [5] above, wherein

the explanatory variables further include the level of a factor selected from the group consisting of creatinine and cystatin C, and
$X_1$ to $X_n$ represent variables for the levels of D- and L-amino acids, creatinine or cystatin C selected by the regression analysis.

[7] The method according to any one of [1] to [5] above, wherein

the explanatory variables are the standardized values for the logarithm of the D- and L-amino acid levels,
the response variables are the standardized values of the logarithm of the glomerular filtration rates, and
the standardized values for the logarithm of the D- and L-amino acid levels selected by the regression analysis are applied to $X_1$ to $X_n$.

[8] The method according to [7] above, wherein

the explanatory variables further include the standardized level of the logarithm of the level of a factor selected from the group consisting of creatinine and cystatin C, and

$X_1$ to $X_n$ represent variables for the levels of D- and L-amino acids, creatinine or cystatin C selected by the regression analysis, the standardized levels of the logarithm of the levels of D- and L-amino acids, creatinine or cystatin C selected by the regression analysis being applied to $X_1$ to $X_n$.

[9] The method according to any one of [1] to [8] above, wherein $X_1$ to $X_n$ include at least the variable of the level of a factor selected from the group consisting of D-serine, D-alanine, D-proline and D-asparagine.

[10] The method according to any one of [1] to [9] above, wherein the glomerular filtration rate of the arbitrary subjects are the glomerular filtration rates calculated by inulin clearance, creatinine clearance, $^{51}$Cr-EDTA clearance, $^{125}$I-sodium iotalamate clearance, $^{99m}$Tc-DTPA clearance, sodium thiosulfate clearance, iohexol clearance, iodixanol clearance or iotalamate clearance.

[11] The method according to any one of [1] to [10] above, wherein the subject being evaluated is a subject who has been assessed to have suspected kidney disease by a conventional examination method.

[12] The method according to any one of [1] to [11] above, wherein treatment intervention is carried out for a subject being evaluated who has been assessed to have reduced kidney function.

[13] The method according to [12] above, wherein the treatment intervention is selected from the group consisting of lifestyle habit improvement, dietary guidance, blood pressure management, anemia management, electrolyte management, uremia management, blood sugar level management, immune management and lipid management.

[14] The method according to [12] or [13] above, wherein the treatment intervention includes administration to a subject of one or more drugs selected from the group consisting of diuretic drugs, calcium antagonists, angiotensin converting enzyme inhibitors, angiotensin receptor antagonists, sympatholytic drugs, SGLT2 inhibitors, sulfonylurea drugs, thiazolidine drugs, biguanide drugs, $\alpha$-glucosidase inhibitors, glinide drugs, insulin formulations, NRF2 activators, immunosuppressive agents, statins, fibrates, anemia treatments, erythropoietin formulations, HIF-1 inhibitors, iron agents, electrolyte regulators, calcium receptor agonists, phosphorus adsorbents, uremic toxin adsorbents, DPP4 inhibitors, EPA formulations, nicotinic acid derivatives, cholesterol nicotinic acid derivatives, cholesterol transporter inhibitors and PCSK9 inhibitors.

[15] A system for estimating kidney function of an subject being evaluated, the system including a storage unit, an analytical measurement unit, a data processing unit and an output unit, wherein:

the storage unit stores the following formula (II):

$$Y = a_1 \cdot X_1 + a_2 \cdot X_2 + \ldots + a_n \cdot X_n + b \quad (II)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (II) is predetermined the regression analysis using D- and L-amino acid levels in biological samples of arbitrary subjects as the explanatory variables and glomerular filtration rates of the arbitrary subjects as the response variables,

the analytical measurement unit quantifies the levels of D- and L-amino acids in a biological sample from the subject being evaluated,
the data processing unit inputs the D- and L-amino acid levels in the biological sample of the subject being evaluated into formula (II) stored in the storage unit to calculate the value of Y and estimates the kidney function of the subject being evaluated, based on the value of Y, and
the output unit outputs information regarding the estimated kidney function of the subject being evaluated.

[16] The system according to [15] above, wherein the data processing unit inputs the D- and L-amino acid levels in the biological sample of the subject being evaluated into formula (II) stored in the storage unit to calculate the value of Y, and estimates the glomerular filtration rate of the subject being evaluated based on the value of Y, thereby estimating the kidney function of the subject being evaluated.

[17] The system according to [15] or [16] above, wherein the biological sample is blood, plasma or serum.

[18] The system according to any one of [15] to [17] above, wherein formula (II) with a correlation coefficient of R $\geq$ 0.5,

is used.

[19] The system according to any one of [15] to [17] above, wherein formula (II) with a correlation coefficient of $R \geq 0.8$, is used.

[20] The system according to any one of [15] to [19] above, wherein the explanatory variables further include the level of a factor selected from the group consisting of creatinine and cystatin C, and

$X_1$ to $X_n$ represent variables for the levels of D- and L-amino acids, creatinine or cystatin C selected by the regression analysis.

[21] The system according to any one of [15] to [19] above, wherein the explanatory variables are the standardized values for the logarithm of the D- and L-amino acid levels,

the response variables are the standardized values of the logarithm of the glomerular filtration rates, and

the standardized values for the logarithm of the D- and L-amino acid levels selected by the regression analysis are applied to $X_1$ to $X_n$.

[22] The system according to [21] above, wherein

the explanatory variables further include the standardized level of the logarithm of the level of a factor selected from the group consisting of creatinine and cystatin C, and

$X_1$ to $X_n$ represent variables for the levels of D- and L-amino acids, creatinine or cystatin C selected by the regression analysis, the standardized levels of the logarithm of the levels of D- and L-amino acids, creatinine or cystatin C selected by the regression analysis being applied to $X_1$ to $X_n$.

[23] The system according to any one of [15] to [22] above, wherein $X_1$ to $X_n$ include at least the variable of the level of a factor selected from the group consisting of D-serine, D-alanine, D-proline and D-asparagine.

[24] The system according to any one of [15] to [23] above, wherein the glomerular filtration rates of the arbitrary subjects are the glomerular filtration rates calculated by inulin clearance, creatinine clearance, $^{51}$Cr-EDTA clearance, $^{125}$I-sodium iotalamate clearance, $^{99m}$Tc-DTPA clearance, sodium thiosulfate clearance, iohexol clearance, iodixanol clearance or iotalamate clearance.

[25] The system according to any one of [15] to [24] above, wherein the subject being evaluated is a subject being evaluated who has been assessed to have suspected kidney disease by a conventional examination method.

[ADVANTAGEOUS EFFECTS OF INVENTION]

The D- and L-amino acid levels in blood used for the method for estimating kidney function of the invention correlate better with GFR (such as inulin clearance) than conventionally used blood creatinine or cystatin C levels. According to the invention it is therefore possible to use a biological sample to conveniently estimate kidney function of a subject being evaluated.

BRIEF DESCRIPTION OF DRAWINGS

Fig. 1 is a block diagram of a system for estimating kidney function according to the invention.

Fig. 2 is a flow chart showing an example of operation for estimating kidney function by the program of the invention. The tables in Fig. 3-1 and Fig. 3-2 show the levels of D- and L-amino acids (nmol/mL), cystatin C (mg/dL) and creatinine (mg/dL) in the blood, and the glomerular filtration rates based on inulin clearance (mL/min/1.73 m$^2$) of 15 healthy volunteers (#1 to 15) and 11 chronic kidney disease (CKD) patients (#21 to 31).

Fig. 4 shows results for the formula for estimating kidney function obtained by regression analysis in Example 1.

Fig. 5 shows results for the formula for estimating kidney function obtained by regression analysis in Example 1.

Fig. 6 shows results for the formula for estimating kidney function obtained by regression analysis in Example 1.

Fig. 7 shows results for the formula for estimating kidney function obtained by regression analysis in Example 1.

Fig. 8 shows results for the formula for estimating kidney function obtained by regression analysis in Example 1.

Fig. 9-1 shows results for the formula for estimating kidney function obtained by regression analysis in Example 1.

Fig. 9-2 shows results for the formula for estimating kidney function obtained by regression analysis in Example 1.

Fig. 9-3 shows results for the formula for estimating kidney function obtained by regression analysis in Example 1.

Fig. 10 shows the levels of D-amino acids (nmol/mL) in the blood, and the glomerular filtration rates based on inulin clearance (mL/min/1.73 m$^2$) of 15 healthy volunteers (#1 to 15) and 11 chronic kidney disease (CKD) patients (#21 to 31), similar to Fig. 3.

Fig. 11 shows results for the formula for estimating kidney function obtained by regression analysis in Example 2.

DESCRIPTION OF EMBODIMENTS

The present invention relates to a method for estimating kidney function of a subject being evaluated, and to a system for estimating kidney function of a subject being evaluated, based on D- and L-amino acid levels in a biological sample.

According to one embodiment, the invention provides a method for estimating kidney function of a subject being evaluated, wherein the method includes:

a step of estimating the kidney function of the subject being evaluated based on the value of Y calculated from the levels of D- and L-amino acids in a biological sample from the subject being evaluated, using the following formula (I):

$$Y = a_1 \cdot X_1 + a_2 \cdot X_2 + \ldots + a_n \cdot X_n + b \text{ (I)}$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (I) is predetermined by the regression analysis using D- and L-amino acid levels in biological samples of arbitrary subjects as the explanatory variables and glomerular filtration rates of the arbitrary subjects as the response variables. The method may be used to provide preliminary data for diagnosis by a physician, and may therefore be considered a preliminary method to diagnosis or a diagnostic aid method.

As used herein, "arbitrary subjects" mean subjects being measured both for levels of D- and L-amino acids (often including creatinine and/or cystatin C levels) in a biological sample, and glomerular filtration rate, used for calculating the regression formula, and it may also include "a subject being evaluated". The number of arbitrary subjects used to calculate the regression formula is preferably a number sufficient to calculate a statistically significant regression formula, and for the purpose of the invention a number of, for example, 3, 5, 10, 20, 30, 50, 100 or greater may be used. The arbitrary subjects measured for calculating the regression formula preferably include a subject with kidney disease and a healthy subject (such as a non-kidney disease subject).

As used herein, a "biological sample" is a sample derived from an organism, and it may be blood, plasma, serum, saliva, urine, ascites fluid, amnionic fluid, lymph, semen, spinal fluid, nasal discharge, sweat, milk or tears, although blood, plasma or serum is preferred as the biological sample for the invention.

As used herein, "D- and L-amino acids" refer to amino acids that are constituents of "D-form" and "L-form" proteins, as well as amino acids that include glycine which has no stereoisomer, and specifically they include glycine, D, L-alanine, D, L-histidine, D, L-isoleucine, D, L-alloisoleucine, D, L-leucine, D, L-lysine, D, L-methionine, D, L-phenylalanine, D, L-threonine, D, L-allothreonine, D, L-tryptophan, D, L-valine, D, L-arginine, D, L-cysteine, D, L-glutamine, D, L-proline, D, L-tyrosine, D, L-aspartic acid, D, L-asparagine, D, L-glutamic acid and D, L-serine. Since D, L-cysteine in a biological sample is oxidized *ex vivo* and converted to D, L-cystine, one embodiment of the invention allows measurement of D, L-cystine instead of D, L-cysteine, calculating the level of D, L-cysteine in the biological sample.

As used herein, "glomerular filtration capacity" refers to the ability of the glomeruli to filter blood. According to one aspect, it is represented as the glomerular filtration rate (GFR), but the glomerular filtration capacity is not limited to the actual glomerular filtration rate and may be determined in arbitrary units. As an example, the blood D- and L-amino acid levels may be represented as the glomerular filtration capacity either directly or after correction by an arbitrary value depending on the case. According to the invention, the glomerular filtration capacity may be the glomerular filtration capacity corrected for body surface area, or it may be the glomerular filtration capacity without correction for body surface area. Because glomerular filtration capacity requirement differs depending on physical size, correction for body surface area is often used for comparison, statistical processing or screening diagnosis.

The glomerular filtration rate is represented in units of "mL/min", indicating the liquid volume filtered per minute from blood by the glomeruli. Since the glomerular filtration rate requirement varies according to physical size, it is corrected to the glomerular filtration rate per standard body surface area of 1.73 $m^2$ for statistical processing, comparison and screening diagnosis, and units of "mL/min/1.73 $m^2$" are used. While it is common to use the body surface area-corrected value for comparison and screening diagnosis of kidney function, the value without body surface area correction is used for individual kidney function diagnosis and dosage determination of kidney excretion drugs. The glomerular filtration rates of arbitrary subjects used as the response variables of the invention may be calculated from inulin clearance, or it may be calculated from creatinine clearance, $^{51}$Cr-EDTA clearance, $^{125}$I-sodium iotalamate clearance, $^{99m}$Tc-DTPA clearance, sodium thiosulfate clearance, iohexol clearance, iodixanol clearance or iotalamate clearance.

The D- and L-amino acids used as markers for the invention are strictly regulated in the tissues and blood, but D- and L-amino acid levels in blood vary in cases of kidney damage.

The "D- and L-amino acid levels in a biological sample" referred to herein may be the D- and L-amino acid levels in a specified amount of biological sample, or it may be their concentrations. The D- and L-amino acid levels in a biological sample are measured as the amounts in a harvested biological sample that has been treated by centrifugal separation, sedimentation separation or other pretreatment for analysis. Therefore, the D- and L-amino acid levels in the biological sample can be measured as the amount in a blood sample derived from sampled whole blood, serum or plasma, for example. For analysis using HPLC, as one example, the D- and L-amino acid levels in a predetermined amount of blood are represented in a chromatogram, and the peak heights, areas and shapes may be quantified by analysis based on standard sample comparison and calibration. It is possible to measure the D- and L-amino acid concentrations in blood by comparison with samples of known D- and L-amino acid concentrations, allowing the D- and L-amino acid concentrations in blood to be used as the D- and L-amino acid levels in blood. With an enzyme method, the amino acid concentration can be calculated by quantitative analysis using a standard calibration curve.

The D- and L-amino acid levels may be measured by any method, such as chiral column chromatography, or measurement using an enzyme method, or quantitation by an immunological method using a monoclonal antibody that distinguishes between optical isomers of amino acids. Measurement of the D- and L-amino acid levels in a sample according to the invention may be carried out using any method well known to those skilled in the art. Examples include chromatographic and enzyme methods (Y. Nagata et al., Clinical Science, 73 (1987), 105. Analytical Biochemistry, 150 (1985), 238., A. D'Aniello et al., Comparative Biochemistry and Physiology Part B, 66 (1980), 319. Journal of Neurochemistry, 29 (1977), 1053., A. Berneman et al., Journal of Microbial & Biochemical Technology, 2 (2010), 139., W. G. Gutheil et al., Analytical Biochemistry, 287 (2000), 196., G. Molla et al., Methods in Molecular Biology, 794 (2012), 273., T. Ito et al., Analytical Biochemistry, 371 (2007), 167.), antibody methods (T. Ohgusu et al., Analytical Biochemistry, 357 (2006), 15), gas chromatography (GC) (H. Hasegawa et al., Journal of Mass Spectrometry, 46 (2011), 502., M. C. Waldhier et al., Analytical and Bioanalytical Chemistry, 394 (2009), 695., A. Hashimoto, T. Nishikawa et al., FEBS Letters, 296 (1992), 33., H. Bruckner and A. Schieber, Biomedical Chromatography, 15 (2001), 166., M. Junge et al., Chirality, 19 (2007), 228., M. C. Waldhier et al., Journal of Chromatography A, 1218 (2011), 4537), capillary electrophoresis methods (CE) (H. Miao et al., Analytical Chemistry, 77 (2005), 7190., D. L. Kirschner et al., Analytical Chemistry, 79 (2007), 736., F. Kitagawa, K. Otsuka, Journal of Chromatography B, 879 (2011), 3078., G. Thorsen and J. Bergquist, Journal of Chromatography B, 745 (2000), 389.), and high-performance liquid chromatography (HPLC) (N. Nimura and T. Kinoshita, Journal of Chromatography, 352 (1986), 169., A. Hashimoto et al., Journal of Chromatography, 582 (1992), 41., H. Bruckner et al., Journal of Chromatography A, 666 (1994), 259., N. Nimura et al., Analytical Biochemistry, 315(2003), 262., C. Muller et al., Journal of Chromatography A, 1324 (2014), 109., S. Einarsson et al., Analytical Chemistry, 59 (1987), 1191., E. Okuma and H. Abe, Journal of Chromatography B, 660 (1994), 243., Y. Gogami et al., Journal of Chromatography B, 879 (2011), 3259., Y. Nagata et al., Journal of Chromatography, 575 (1992), 147., S. A. Fuchs et al., Clinical Chemistry, 54 (2008), 1443., D. Gordes et al., Amino Acids, 40 (2011), 553., D. Jin et al., Analytical Biochemistry, 269 (1999), 124., J. Z. Min et al., Journal of Chromatography B, 879 (2011), 3220., T. Sakamoto et al., Analytical and Bioanalytical Chemistry, 408 (2016), 517., W. F. Visser et al., Journal of Chromatography A, 1218 (2011), 7130., Y. Xing et al., Analytical and Bioanalytical Chemistry, 408 (2016), 141., K. Imai et al., Biomedical Chromatography, 9 (1995), 106., T. Fukushima et al., Biomedical Chromatography, 9 (1995), 10., R. J. Reischl et al., Journal of Chromatography A, 1218 (2011), 8379., R. J. Reischl and W. Lindner, Journal of Chromatography A, 1269 (2012), 262., S. Karakawa et al., Journal of Pharmaceutical and Biomedical Analysis, 115 (2015), 123.).

The separative analysis system for optical isomers according to the invention may be a combination of multiple separative analysis methods. More specifically, the D-/L-amino acid level in a sample can be measured using an optical isomer analysis method comprising a step of passing a sample containing a component with optical isomers through a first column filler as the stationary phase, together with a first liquid as the mobile phase, to separate the components in the sample, a step of separately holding each of the components in the sample in a multi loop unit, a step of passing each of the components in the sample that are separately held in the multi loop unit through a flow channel in a second column filler having an optically active center, as the stationary phase, together with a second liquid as the mobile phase, to separate the optical isomers among each of the sample components, and a step of detecting the optical isomers in each of the sample components (Japanese Patent No. 4291628). In HPLC analysis, D- and L-amino acids are sometimes pre-derivatized with a fluorescent reagent such as o-phthalaldehyde (OPA) or 4-fluoro-7-nitro-2,1,3-benzoxadiazole (NBD-F), or diastereomerized using an agent such as N-tert-butyloxycarbonyl-L-cysteine (Boc-L-Cys) (Hamase, K. and Zaitsu, K., Bunseki Kagaku, Vol.53, 677-690(2004)). Alternatively, the D-amino acids may be measured by an immunological method using a monoclonal antibody that distinguishes optical isomers of amino acids, such as a monoclonal antibody that specifically binds to D- and L-amino acids. When the total of the D-form and L-form is to be used as the marker it is not necessary to separate the D-form and L-form, allowing the amino acids to be analyzed without separating the D-form and L-form. In such cases as well, separation and quantitation may be carried out using an enzyme method, antibody method, GC, CE or HPLC.

The following formula (I):

$$Y = a_1 \cdot X_1 + a_2 \cdot X_2 + \ldots + a_n \cdot X_n + b \quad (I)$$

[where

a_1 to a_n represent constants obtained by regression analysis,
$X_1$ to $X_n$ represent variables for the D- and L-amino acid levels selected by the regression analysis, and
b represents a constant obtained by the regression analysis],

is predetermined by regression analysis using D- and L-amino acid levels in biological samples of arbitrary subjects as the explanatory variables and glomerular filtration rates of the arbitrary subjects as the response variables. According to the invention it is possible to use formula (I) to estimate kidney function of a subject being evaluated.

As used herein, "predetermined" means determined before the point at which the Y value is calculated based on levels of D- and L-amino acids in a biological sample from a subject being evaluated (often including creatinine and/or cystatin C levels). Therefore, the point at which formula (I) is determined is not particularly restricted so long as it is before the point at which the Y value is calculated. Formula (I) used for the invention may be a formula determined by the person who calculates the value of Y, or it may be a formula determined by a third party who does not calculate the value of Y. That is, the invention encompasses cases of calculating the value of Y using any formula within the range of formula (I) of the invention and carrying out the step of estimating the kidney function of the subject being evaluated, based on the obtained value of Y.

As used herein, "regression analysis" refers to a method of estimating a formula representing the relationship between an explanatory variable (also known as "independent variable") and a response variable (also known as "dependent variable") in a statistical manner, and for example, it is a solution method by regression using the least square method, moving average method or kernel method. Regression analysis is a well-known method and any regression analysis may be used for the invention. Regression used in regression analysis for the invention may be linear regression, or it may be nonlinear regression (such as "n"th polynomial regression analysis). The regression used for the invention may be simple linear regression or multiple regression. According to the invention, a formula for determining the value of Y, is determined by regression analysis using levels of D- and L-amino acids (often including creatinine and/or cystatin C levels) in biological samples of arbitrary subjects as the explanatory variables, and glomerular filtration rates of the arbitrary subjects as the response variables.

As used herein, "a_1 to a_n" represents the constants obtained by regression analysis using levels of D- and L-amino acids (often including creatinine and/or cystatin C levels) in biological samples of arbitrary subjects as the explanatory variables, and glomerular filtration rates of the arbitrary subjects as the response variables.

As used herein, "$X_1$ to $X_n$" represents the variables of D- and L-amino acid levels (often including creatinine and/or cystatin C levels) selected by regression analysis using levels of D- and L-amino acids (often including creatinine and cystatin C levels) in biological samples of arbitrary subjects as the explanatory variables, and glomerular filtration rates of the arbitrary subjects as the response variables, and depending on the regression analysis employed, they may be expressed as exponential variables of a first-order function, second-order function, third-order function ... nth order function (where n is a natural number). As used herein, the "n" subscript in "$a_n$" and "$X_n$" represents a natural number of $1 \leq n \leq 45$, as numbers assigned to distinguish factors selected from the group consisting of D- and L-amino acids, and sometimes creatinine and cystatin C, used for the invention. Therefore, "n" is equivalent to the number of D- and L-amino acids, creatinine and cystatin C selected by regression analysis.

As used herein, "b" represents the constant (also referred to as intercept) obtained by regression analysis using levels of D- and L-amino acids (often including creatinine and/or cystatin C levels) in biological samples of arbitrary subjects as the explanatory variables, and glomerular filtration rates of the arbitrary subjects as the response variables.

Formula (I) derived by regression analysis is preferably a formula with correlation coefficient $R \geq 0.5$, more preferably correlation coefficient $R \geq 0.6$, even more preferably correlation coefficient $R \geq 0.7$ and most preferably correlation coefficient $R \geq 0.8$.

Since the blood creatinine level to be used for comparison with the invention is significantly affected by the amount of muscle from which it is derived, sports athletes, acromegaly patients and persons that have ingested large amounts of meat will exhibit higher values, while patients suffering from neuromuscular disease (such as muscular dystrophy), emaciation, prolonged bed rest, frailty, sarcopenia, locomotive syndrome or amputation, or persons that have restricted their protein intake, will exhibit lower values, and therefore accurate kidney function cannot be reflected. Moreover, since blood creatinine levels have been found to have circadian variation of about 10%, with higher values in the morning, care must also be taken in that regard. Since blood cystatin C level increases sharply compared to blood creatinine level with moderate reduction in kidney function, it is considered to be advantageous for discovering early impaired kidney function. However, it is also known that the levels are affected by the use of steroids and cyclosporins, and by patient conditions such as diabetes, hyperthyroidism, inflammation, hyperbilirubinemia and hypertriglyceridemia. For examination of kidney disease, therefore, it is necessary to make a comprehensive diagnosis in combination with other markers such as urea nitrogen (BUN) and urine proteins.

The accuracy of the glomerular filtration rate determined based on conventional kidney function markers such as blood creatinine levels is low, and while accurate glomerular filtration rate measurement is possible based on the gold standard of inulin clearance, it involves complex methods and a burden on patients and health care professionals, and is therefore limited in its practicality. The method of determining glomerular filtration capacity according to the invention at least allows the glomerular filtration capacity to be more accurately determined than by blood creatinine levels, and even allows glomerular filtration capacity to be determined more accurately than by blood cystatin C levels. When analysis was made in correlation with inulin clearance in groups classified according to glomerular filtration rate, D-serine exhibited higher correlation coefficient R values and higher correlation with inulin clearance, than both blood cystatin C and creatinine levels across all the groups. Future experiments should be conducted to determine accuracy, but it has the potential to equal or even surpass the performance obtained by the glomerular filtration rate determining method based on inulin clearance, which is the international standard for measurement. According to another aspect of the invention, therefore, it is possible to use blood D- and L-amino acid levels as a substitute marker for inulin clearance. A substitute marker is a marker whose relationship with a final evaluation can be scientifically proven. Being a substitute marker for inulin clearance, therefore, means that glomerular filtration rate can be determined based on D- and L-amino acid levels instead of using a GFR determining method based on inulin clearance, as a result of having used blood D- and L-amino acid levels to statistically demonstrate a relationship with evaluation based on inulin clearance.

While it is not our intention to be limited to any particular theory, D- and L-amino acids have the advantage of not being affected by muscle mass and thus not requiring correction for physical size as is necessary for blood creatinine. According to one embodiment of the invention, the method for estimating kidney function is characterized by not correcting for one or more physical size-related factors selected from the group consisting of gender, age and muscle mass.

The explanatory variable used for calculation of formula (I) according to one embodiment of the invention may be the "D- and L-amino acid level", or the level of a factor selected from the group consisting of creatinine and cystatin C. In this case, $X_1$ to $X_n$ represent the variables for levels of D- and L-amino acids, creatinine or cystatin C selected by the regression analysis.

According to another embodiment of the invention, the explanatory variables for calculation of formula (I) are the standardized values for the logarithm of the D- and L-amino acid levels in biological samples of arbitrary subjects, while the response variables may be the standardized values of the logarithm of the glomerular filtration rates for the arbitrary subjects. In this case, the standardized values for the logarithm of the D- and L-amino acid levels selected by regression analysis are applied for $X_1$ to $X_n$ in formula (I).

As used herein, "logarithm of the D- and L-amino acid levels" is the value for the D- and L-amino acid levels converted to the natural logarithm. As used herein, "standardized value for the logarithm of the D- and L-amino acid levels" is the value calculated using the following formula (A):

[Mathematical Formula 1]

$$\text{Standardized value for the logarithm of the D- and L-amino acid levels} = \frac{X - \overline{X}}{s_x}$$

$\times$ : Logarithm of D- and L-amino acid levels for arbitrary subject

$\overline{x}$ : Average of logarithm of D- and L-amino acid levels for parent population

$s_x$ : Standard deviation of logarithm of D- and L-amino acid levels for parent population

The "parent population" here is a group including all of the arbitrary subjects quantified for regression analysis.

As used herein, "logarithm of the glomerular filtration rate" is the value of the glomerular filtration rate converted to the natural logarithm. The "standardized value of the logarithm of the glomerular filtration rate" is the value calculated using the following formula (B):

[Mathematical Formula 2]

$$\text{Standardized value of the logarithm of the glomerular filtration rate} = \frac{y - \bar{y}}{s_y}$$

$\underline{y}$ : Logarithm of glomerular filtration rate for arbitrary subject

$\bar{y}$ : Average of logarithm of glomerular filtration rate for parent population

$s_y$: Standard deviation of logarithm of glomerular filtration rate for parent population

For conversion from the value of Y obtained for this embodiment to an estimated value for the glomerular filtration rate, the opposite order from formula (B) can be used, i.e. by multiplying the Y value by the standard deviation of the logarithm of the glomerular filtration rate for the parent population and adding the average value of the logarithm of the glomerular filtration rate for the parent population, and then transforming the obtained value to an exponent.

According to another aspect, the explanatory variables may further include the standardized level of the logarithm of the amount of a factor selected from the group consisting of creatinine and cystatin C, in addition to D- and L-amino acids (the standardized levels of the logarithms of the amounts of creatinine and cystatin C being calculated by the same method as formula (A) above). In this case, $X_1$ to $X_n$ of formula (I) represent variables for the levels of D- and L-amino acids, creatinine or cystatin C selected by the regression analysis, the standardized levels of the logarithms of the levels of D- and L-amino acids, creatinine or cystatin C selected by the regression analysis being applied to $X_1$ to $X_n$.

According to one embodiment of the invention, $X_1$ to $X_n$ include at least the variable of the level of a factor selected from the group consisting of D-serine, D-alanine, D-proline and D-asparagine. This is preferred for higher correlation of formula (I) of the invention with the glomerular filtration rate.

According to one aspect of the invention, the kidney function of an subject being evaluated can be estimated based on the value of Y obtained by inserting blood D- and L-amino acid levels of an subject being evaluated into the formula derived from the correlation between the inulin clearance and blood D- and L-amino acid levels of the arbitrary subject, and a corresponding table or graph. The correlation between inulin clearance and D- and L-amino acid levels has been shown to be higher than the correlation between inulin clearance and blood creatinine level, and therefore glomerular filtration capacity estimated by substituting D- and L-amino acid level of an subject being evaluated into a formula, or a corresponding table or graph, derived from the correlation between inulin clearance and blood D- and L-amino acid levels, is more accurate than the conventional glomerular filtration rate estimated from blood creatinine level. The inulin clearance used for correlation analysis may be the inulin clearance after correction for body surface area or the inulin clearance before correction for body surface area. Glomerular filtration capacity before or after correction for body surface area may be selected according to the need. A correspondence table derived from the correlation between inulin clearance and blood D- and L-amino acid levels may list values for glomerular filtration capacity corresponding to D- and L-amino acid levels, or it may list kidney functions corresponding to numerical ranges, i.e. categories for severity of kidney disease.

The severity categories for chronic kidney disease (CKD) are the 6 levels of G1, G2, G3a, G3b, G4 and G5, according to the numerical ranges for glomerular filtration rate. Specifically, the definitions are normal or high value for 90 mL/min/1.73 $m^2$ or greater (G1), normal or mildly low for 60 to 89 mL/min/1.73 $m^2$ (G2), mildly to moderately low for 45 to 59 mL/min/1.73 $m^2$ (G3a), moderately to severely low for 30 to 44 mL/min/1.73 $m^2$ (G3b), severely low for 15 to 29 mL/min/1.73 $m^2$ (G4) and end-stage kidney disease for less than 15 mL/min/1.73 $m^2$ (G5) (Japanese Society of Nephrology Guidelines).

Various formulas have been devised for blood creatinine level or cystatin C level which is observed to correlate with physical size, estimating the glomerular filtration rate by correcting for race, age and gender using large-scale patient data. The major estimation formulas for glomerular filtration rate are the Cockcroft-Gault formula, MDRD formula and CKD-EPI formula, and currently the estimation formula (eGFR) used for routine examination for Japanese is the following.

[Mathematical Formula 3]

$$\text{eGFR creat} \quad \begin{array}{l} \text{Male mL/minute/1.73}\,m^2 = 194 \times Cr^{-1.094} \times Age^{-0.287} \\ \text{Female mL/minute/1.73}\,m^2 = 194 \times Cr^{-1.094} \times Age^{-0.287} \times 0.739 \end{array}$$

However, the eGFR determined in this manner is a marker created for health examination screening or for convenient evaluation in epidemiologic research for comparison of numerous subjects, with the values being intended to be calculated with correction to average physical size, and therefore it is still recommended to use inulin clearance for accurate evaluation of kidney function for individual patients including excessively lean elderly (Japanese Society of Nephrology Guidelines).

The present invention allows estimation of kidney function, such as the severity of kidney disease, based on the determined Y value. As an example, the invention allows categorization of severity on the 6 levels of G1, G2, G3a, G3b, G4 and G5, as classifications for chronic kidney disease patients based on the value of Y. Treatment intervention is used for subjects classified in categories corresponding to G2 to G5. The treatment intervention is selected as appropriate for each category. Treatment intervention is guidance for one or a combination from among lifestyle habit improvement, dietary guidance, blood pressure management, anemia management, electrolyte management, uremia management, blood sugar level management, immune management or lipid management. Lifestyle habit improvement may be a recommendation to stop smoking or to reduce the BMI value to below 25. Dietary guidance may be salt or protein restriction. For blood pressure management, anemia management, electrolyte management, uremic toxin manage, blood sugar level management, immune management or lipid management in particular, treatment may involve administration of a drug. Blood pressure management may involve general management or administration of an antihypertensive drug, to reach below 130/80 mmHg. Antihypertensive drugs include diuretic drugs (thiazide diuretics such as trichlormethiazide, benzylhydrochlorothiazide and hydrochlorothiazide, thiazide-like diuretics such as meticrane, indapamide, tribamide and mefluside, loop diuretics such as furosemide, and potassium-sparing diuretics and aldosterone antagonists such as triamterene, spironolactone and eplerenone), calcium antagonists (dihydropyridine-based antagonists such as nifedipine, amlodipine, efonidipine, cilnidipine, nicardipine, nisoldipine, nitrendipine, nilvadipine, barnidipine, felodipine, benidipine, manidipine, azelnidipine and aranidipine, benzodiazepine-based antagonists, and diltiazem), angiotensin converting enzyme inhibitors (such as captopril, enalapril, acelapril, delapril, cilazapril, lisinopril, benazepril, imidapril, temocapril, quinapril, trandolapril, perindopril and erbumine), angiotensin receptor antagonists (angiotensin II receptor antagonists such as losartan, candesartan, valsartan, telmisartan, olmesartan, irbesartan and azilsartan), and sympatholytic drugs (β-blockers, such as atenolol, bisoprolol, betaxolol, metoprolol, acebutolol, celiprolol, propranolol, nadolol, carteolol, pindolol, nipradilol, amosulalol, arotinolol, carvedilol, labetalol, bevantolol, urapidil, terazosin, prazosin, doxazosin and bunazosin). Erythropoietin formulations, iron agents and HIF-1 inhibitors are used as anemia treatments. Calcium receptor agonists (such as cinacalcet and etelcalcetide) and phosphorus adsorbents are used as electrolyte regulators. Active carbon is used as a uremic toxin adsorbent. Blood glucose level is managed to Hbalc of <6.9%, and in some cases a hypoglycemic agent is administered. Hypoglycemic agents that are used include SGLT2 inhibitors (such as ipragliflozin, dapagliflozin, luseogliflozin, tofogliflozin, canagliflozin and empagliflozin), DPP4 inhibitors (such as sitagliptin phosphate, vildagliptin, saxagliptin, alogliptin, linagliptin, teneligliptin, trelagliptin, anagliptin, omarigliptin), sulfonylurea agents (such as tolbutamide, acetohexamide, chlorpropamide, glyclopyramide, glibenclamide, gliclazide and glimepiride), thiazolidine agents (such as pioglitazone), biguanide agents (such as metformin and buformin), α-glucosidase inhibitors (such as acarbose, voglibose and miglitol), glinide agents (such as nateglinide, mitiglinide and repaglinide), insulin formulations and NRF2 activators (such as bardoxolonemethyl). Immunosuppressive agents (such as steroids, tacrolimus, anti-CD20 antibody, cyclohexamide and mycophenolate mofetil (MMF)) are used for immune management. Lipid management includes management to lower LDL-C to below 120 mg/dL, or in some cases dyslipidemia treatments are used, including statins (such as rosuvastatin, pitavastatin, atorvastatin, cerivastatin, fluvastatin, simvastatin, pravastatin, lovastatin and mevastatin), fibrates (such as clofibrate, bezafibrate, fenofibrate and clinofibrate), nicotinic acid derivatives (such as nicotinic acid derivatives (tocopherol nicotinate, nicomol and niceritrol), cholesterol transporter inhibitors (such as ezetimibe), PCSK9 inhibitors (such as evolocumab) and EPA formulations. All of these drugs may be used as single dosage forms or mixtures. Depending on the degree of kidney function impairment, kidney replacement therapy such as peritoneal dialysis, hemodialysis, continuous hemodialysis filtration, blood apheresis (such as plasma exchange or plasma adsorption) or kidney transplant may also be carried out.

According to another aspect of the invention, the step of estimating the kidney function of the subject being evaluated may be a step of estimating the kidney function of the subject being evaluated by estimating the glomerular filtration rate of the subject being evaluated, based on the Y value.

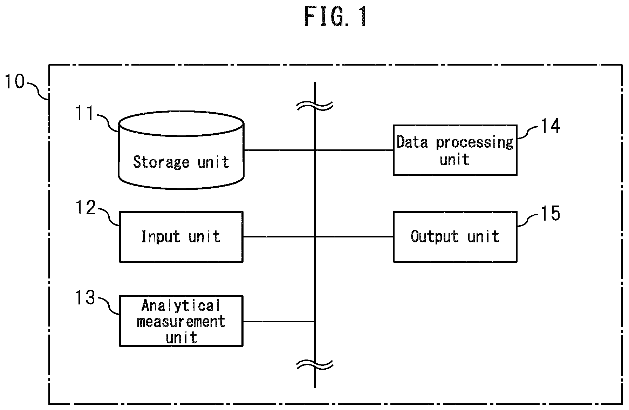

According to another aspect, the invention relates to a system and program for carrying out the aforementioned method for estimating kidney function of a subject being evaluated. Fig. 1 is a block diagram of a sample analysis system of the invention. The sample analysis system 10 shown in Fig. 1 is constructed so as to allow the method for estimating kidney function of a subject being evaluated of the invention to be carried out. The sample analysis system 10 comprises a storage unit 11, an input unit 12, an analytical measurement unit 13, a data processing unit 14 and an output unit 15, and allows analysis of biological samples and output of the kidney function of the subject being evaluated.

More specifically, in the sample analysis system 10 of the invention,

the storage unit 11 stores a predetermined formula (II):

EP 4 083 628 B1

$$Y = a_1 \cdot X_1 + a_2 \cdot X_2 + \ldots + a_n \cdot X_n + b \quad (II)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (II) is predetermined by the regression analysis using D- and L-amino acid levels in biological samples of arbitrary subjects as the explanatory variables and glomerular filtration rates of the arbitrary subjects as the response variables, as inputted through the input unit 12,

the analytical measurement unit 13 quantifies the levels of D- and L-amino acids in a biological sample from the subject being evaluated,

the data processing unit 14 inputs the D- and L-amino acid levels in the biological sample of the subject being evaluated into formula (II) stored in the storage unit to calculate the value of Y and estimates the kidney function of the subject being evaluated, based on the value of Y, and

the output unit 15 outputs information regarding the estimated kidney function of the subject being evaluated. Formula (II) is the same as formula (I) used in the method for estimating kidney function of an subject being evaluated described above, and since the explanation for the method for estimating kidney function of an subject being evaluated is applicable to the system and program for estimating kidney function of an subject being evaluated, it will not be explained in full here.

According to a more preferred aspect, the sample analysis system of the invention may further include a step in which the storage unit 11 stores a threshold value inputted from the input unit 12, and a step in which the data processing unit 14 compares the Y value with the threshold value. Comparison between the Y value and the threshold allows kidney function of the subject being evaluated to be estimated and allows the output unit 15 to output information regarding the estimated kidney function of the subject being evaluated.

The storage unit 11 has a portable storage device which may be a memory device such as a RAM, ROM or flash memory, a fixed disk device such as a hard disk drive, or a flexible disk or optical disk. The storage unit stores data measured by the analytical measurement unit, data and instructions inputted from the input unit, and results of computation processing by the data processing unit, as well as the computer program and database to be used for processing by the information processing equipment. The computer program may be a computer readable recording medium such as a CD-ROM or DVD-ROM, or it may be installed via the internet. The computer program is installed in the storage unit using a commonly known setup program, for example. The storage unit stores the following formula (II):

$$Y = a_1 \cdot X_1 + a_2 \cdot X_2 + \ldots + a_n \cdot X_n + b \quad (II)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (II) is predetermined by regression analysis using D- and L-amino acid levels in biological samples of arbitrary subjects as the explanatory variables and glomerular filtration rates of the arbitrary subjects as the response variables, inputted in advance through the input unit 12. It may also store kidney function categories corresponding to Y values.

The input unit 12 is an interface and also includes operating devices such as a keyboard and mouse. This allows the input unit to input data measured by the analytical measurement unit 13 and instructions for computation processing to be carried out by the data processing unit 14. When the analytical measurement unit 13 is external, for example, the input unit 12 may also include an interface unit allowing input of measured data through a network or storage medium, separately from the operating device.

The analytical measurement unit 13 carries out a step of measuring the D- and L-amino acids in a biological sample. The analytical measurement unit 13 may therefore have a construction allowing separation and measurement of the D-forms and L-forms of amino acids. The amino acids may be analyzed one at a time, or some or all of the amino acid types may be analyzed at once. With no intention to be limitative, the analytical measurement unit 13 may be a chiral chromatography system comprising a sample introduction inlet, an optical resolution column and a detector, for example, and it is preferably a high-performance liquid chromatography system. From the viewpoint of detecting the levels of only specific amino acids, quantitation may be carried out by an enzyme method or immunological method. The analytical measurement unit 13 may be constructed separately from the sample analysis system, and measured data may be inputted through the input unit 12 using a network or storage medium.

The data processing unit 14 can calculate the Y value from the measured D- and L-amino acid levels by inputting them into formula (II) which is predetermined by regression analysis using the D- and L-amino acid levels in biological sample of an arbitrary subjects as the explanatory variables and glomerular filtration rates of the arbitrary subject as the response variables. Formula (II) requires other correction values such as age, body weight, gender or body height, and such information may also be inputted beforehand through the input unit and stored in the storage unit. During calculation of the glomerular filtration rate, the data processing unit may access the information and input it into the formula, or read out a value from the corresponding table or graph, to calculate the glomerular filtration rate. The data processing unit 14 may also determine a kidney disease or kidney function category from the determined glomerular filtration capacity. The data processing unit 14 carries out various computation processing operations on the data measured by the analytical measurement unit 13 and stored in the storage unit 11, based on a program stored in the storage unit. The computation processing is carried out by a CPU in the data processing unit. The CPU includes a functional module that controls the analytical measurement unit 13, input unit 12, storage unit 11 and output unit 15, with the functional module performing various control operations. Each of the units may be constructed by independent integrated circuits, microprocessors and firmware.

The output unit 15 is constructed so as to output the glomerular filtration capacity which is the result of the computation processing by the data processing unit. The output unit 15 may be output means such as a display device with a liquid crystal display that directly displays the computation processing results, or a printer, or it may be an interface unit for output to an external memory unit or output to a network. It may output the Y value either together with the glomerular filtration rate and/or kidney function of the subject being evaluated, or independently.

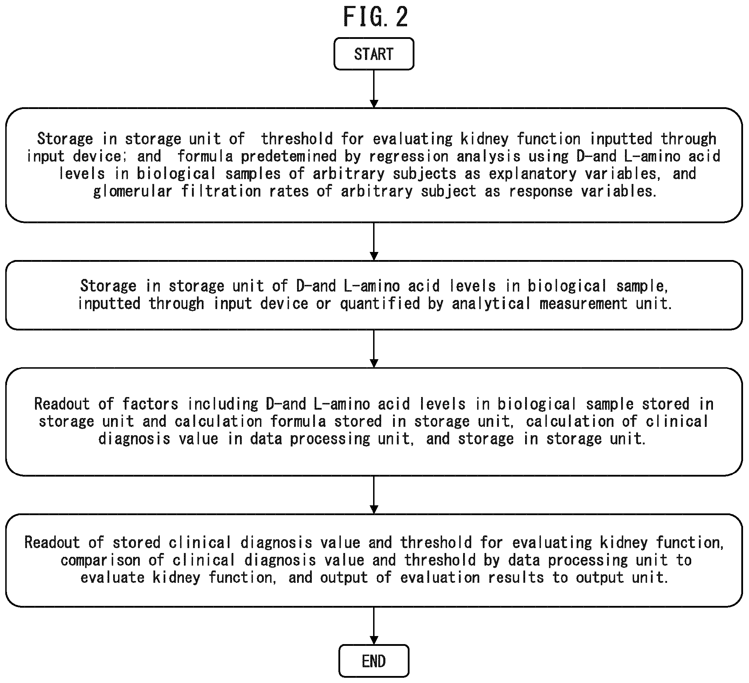

Fig. 2 is a flow chart showing an example of operation for estimating kidney function by the program of the invention. Specifically, the program of the invention is a program that estimates kidney function in an information processing device comprising an input unit, output unit, data processing unit and storage unit. The program of the invention includes a command which causes the information processing device:

to store in the storage unit a D- and L-amino acid level previously inputted through the input device,
to read out the formula (II):

$$Y = a_1 \cdot X_1 + a_2 \cdot X_2 + \ldots + a_n \cdot X_n + b \ (II)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

previously derived by regression analysis using D- and L-amino acid levels in biological samples of arbitrary subjects as the explanatory variables and glomerular filtration rates of the arbitrary subjects as the response variables, and the D- and L-amino acid level previously stored in the storage unit, and determine the Y value in the data processing unit, to store the determined Y value in the storage unit, and
to output the stored Y value to the output unit. The program of the invention may be stored in a storage medium, or it may be provided via electronic transmission such as the internet or a LAN.

When the information processing device comprises an analytical measurement unit, it may include a command for causing the information processing device to take the value for the blood sample measured by the analytical measurement unit and store it in the storage unit, instead of having the D- and L-amino acid level inputted from an input device.

The examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

EXAMPLES

[Example 1]

Group of subjects

Eleven patients were used in a retrospective study, from among a cohort of chronic kidney disease (CKD) patients admitted to Osaka University Hospital, Department of Nephrology for diagnosis and/or treatment between 2016 and 2017. Separately, 15 healthy volunteers of age 20 and older were recruited by the National Institutes of Biomedical Innovation, Health and Nutrition. The test protocol was approved by the ethics committee of each facility, and written informed consent was obtained from all of the subjects.

The information for the healthy subjects and chronic kidney disease patients were as follows.

[Table 1]

| Table 1 Subject baselines | | | |
|---|---|---|---|
| | Healthy subjects, n = 15 | CKD patients (n = 11) | P value |
| Age | 44 (39- 50) | 50 (40- 65) | 0.232 |
| Male percentage (%) | 80 (12) | 45.5 (5) | 0.103 |
| Body height (m) | 1.70 (1.68- 1.75) | 1.63 (1.59- 1.66) | 0.043 |
| Body weight (kg) | 68.9 (61.0- 73.5) | 59.8 (51.5- 66.7) | 0.194 |
| BSA ($m^2$) | 1.80 (1.72- 1.90) | 1.61 (1.54- 1.75) | 0.102 |
| BMI ($kg/m^2$) | 22.6 (21.1- 25.7) | 22.5 (19.3- 24.2) | 0.452 |
| Serum creatinine (mg/dL) | 0.75 (0.68- 0.83) | 1.14 (0.75- 2.59) | 0.069 |
| Serum cystatin C (mg/L) | 0.78 (0.69- 0.84) | 1.14 (0.87- 2.11) | 0.005 |
| Inulin clearance (mL/min/1.73 $m^2$) | 97.0 (94.1- 107.3) | 46.0 (19.8- 66.9) | <0.001 |
| Values represent median (IQR) or %(count) | | | |

Method of measuring inulin clearance

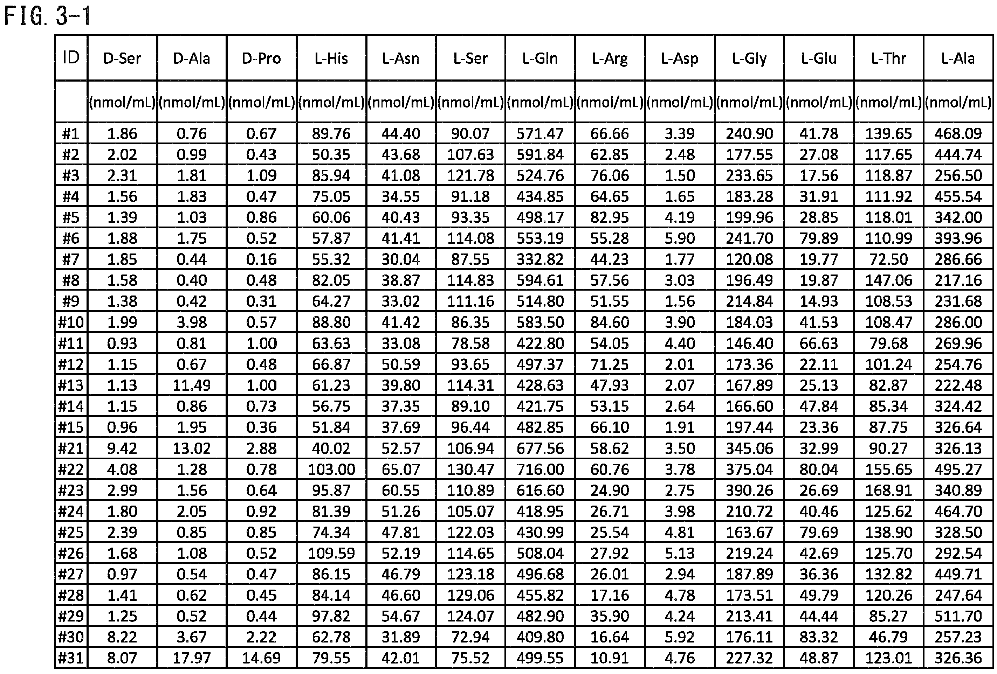

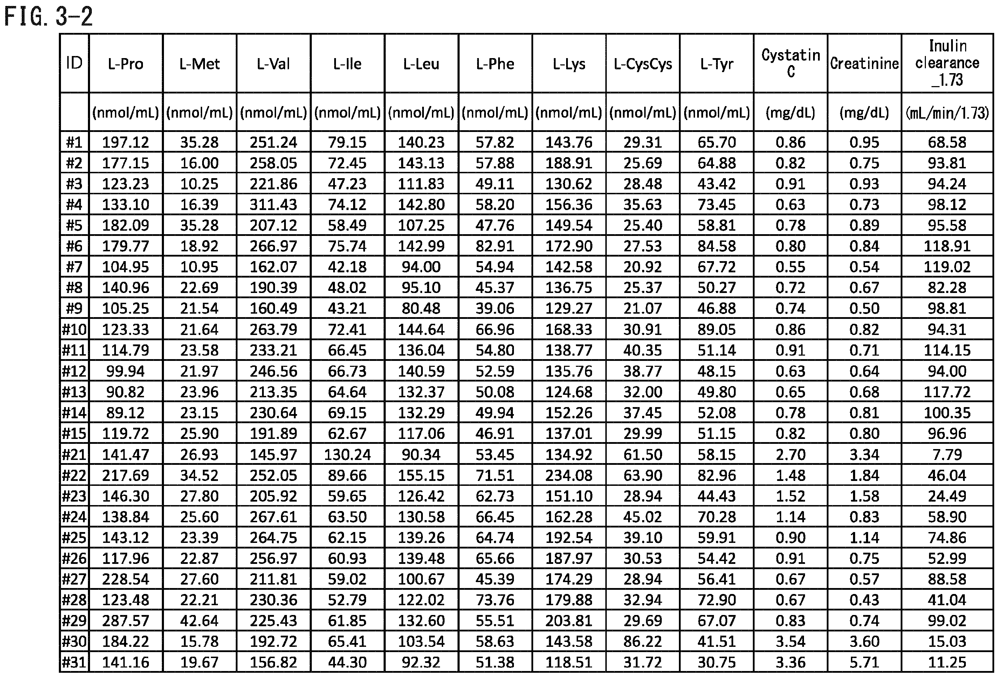

Subject's inulin clearance (Cin) was calculated from the blood and urine inulin concentrations, and urine volume, according to the standard method described in Clin Exp Nephrol 13, 50-54(2009). In brief, 1% inulin (INULEAD injection, Fuji Yakuhin Co., Ltd.) was given by continuous intravenous drip infusion over a period of 2 hours while in a state of fasting, medication postponement and water load, and blood and urine samples were taken at 3 different time points during the period. The subjects ingested 500 mL of water orally at 30 minutes before drip infusion. In order to maintain water load, 60 mL of water was ingested 40, 60 and 90 minutes after starting inulin drip infusion. The initial rate of drip infusion was 300 mL/h for the first 30 minutes, and 100 mL/h for the following 90 minutes. Blood samples were taken at 45, 75 and 105 minutes after the start of inulin drip infusion. The subjects urinated to completely empty bladder at 30 minutes after the start of drip infusion. Urine samples were then taken during the period of 30 to 60 minutes, 60 to 90 minutes and 90 to 120 minutes thereafter. Inulin was measured using an enzyme method. The average of three Cin values was used as Cin (Cin-ST) according to a standard method (Figs. 3-1 and 3-2).

Measurement of blood D- and L-amino acids

Sample preparation

Sample prepare from human plasma was carried out as follows: First a 20-fold volume of methanol was added to and completely mixed with the plasma. After centrifugation, 10 μL of supernatant obtained from the methanol homogenate was transferred to a brown tube and dried under reduced pressure. To the residue there were added 20 μL of 200 mM sodium borate buffer (pH 8.0) and 5 μL of fluorescent labeling reagent (40 mM 4-fluoro-7-nitro-2,1,3-benzooxadiazole (NBD-F) in anhydrous MeCN), and the mixture was then heated at 60°C for 2 minutes. The reaction was suspended by addition of 75 μL of aqueous 0.1% TFA (v/v), and 2 μL of the reaction mixture was supplied to two-dimensional HPLC.

Quantitation of amino acid optical isomers by two-dimensional HPLC

The amino acid optical isomers were quantified using the following two-dimensional HPLC system. NBD derivatives of the amino acids were separated and eluted using a reversed-phase column (KSAA RP, 1.0 mm i.d. × 400 mm; Shiseido Co., Ltd.), in the mobile phase (5 to 35% MeCN, 0 to 20% THF, 0.05% TFA). The column temperature was 45°C and the mobile phase flow rate was 25 μL/min. The separated amino acid fraction was separated off using a multi loop valve, and optically resolved in a continuous manner with a chiral column (KSAACSP-001S, 1.5 mm i.d. × 250 mm; Shiseido Co., Ltd.). The mobile phase used was a MeOH/MeCN mixed solution containing citric acid (0 to 10 mM) or formic acid (0 to 4%), according to the amino acid retention. NBD-amino acids were detected by fluorescence detection at 530 nm using excitation light of 470 nm. The NBD-amino acid retention time was identified from standard amino acid optical isomers and quantified by a calibration curve (Figs. 3-1 and 3-2).

Derivation of formula for estimating kidney function by regression analysis

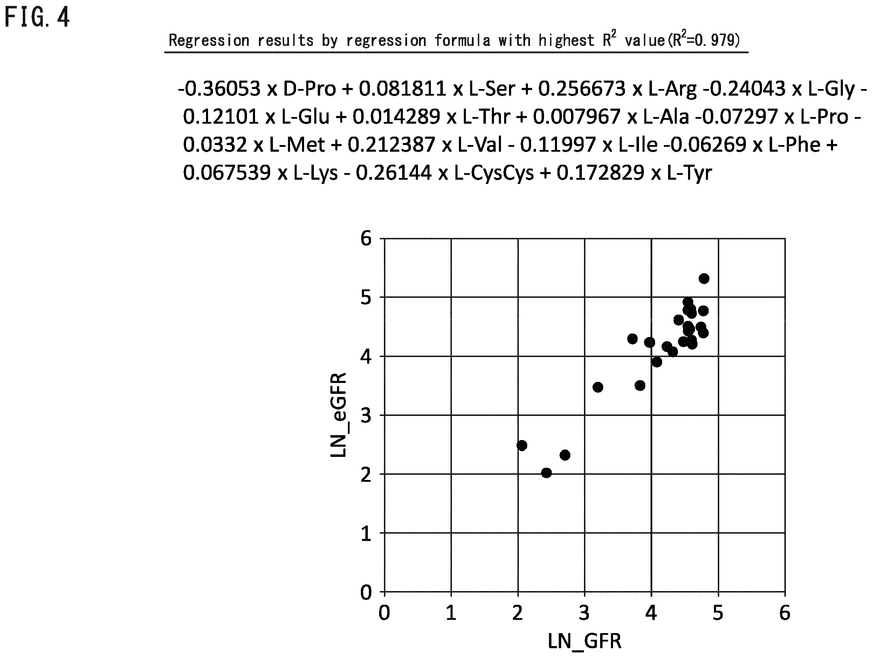

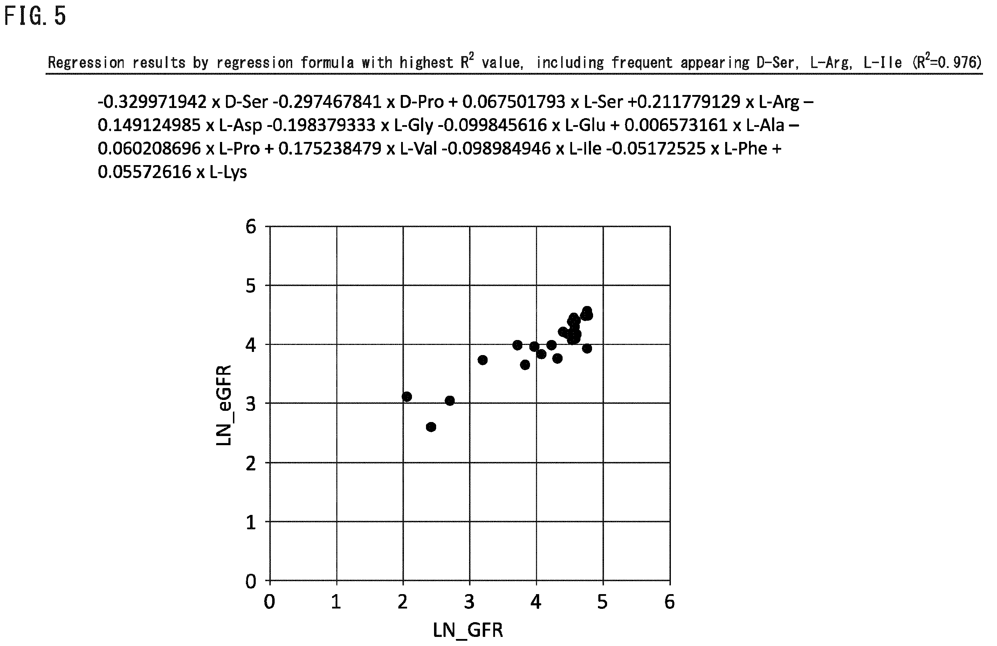

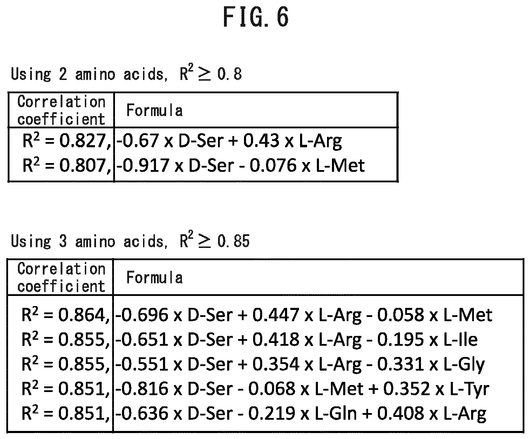

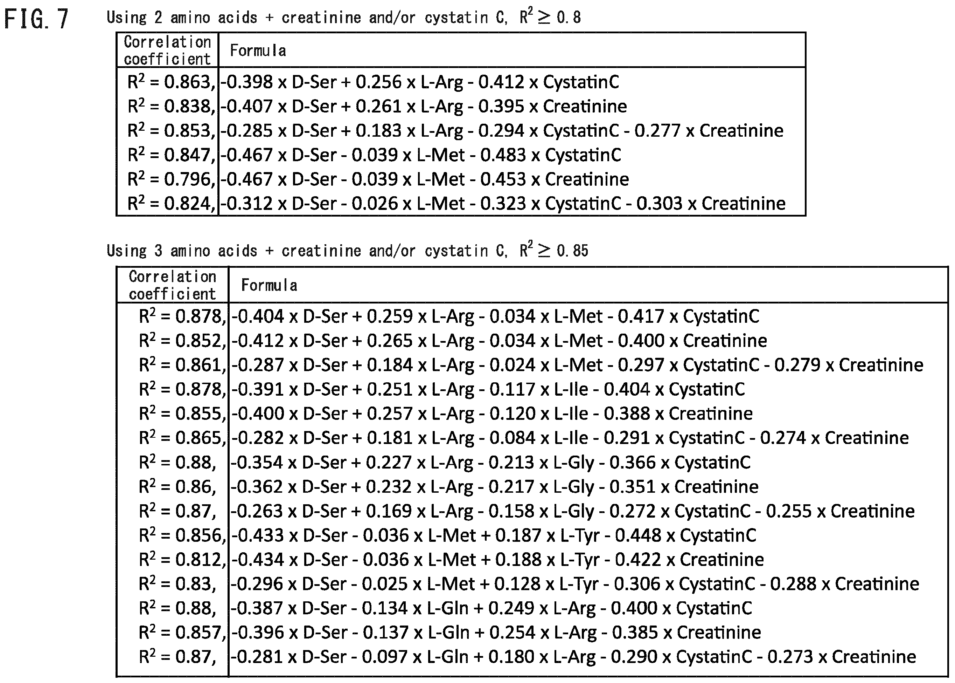

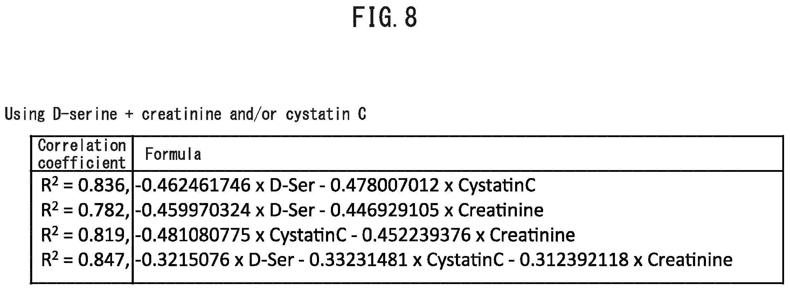

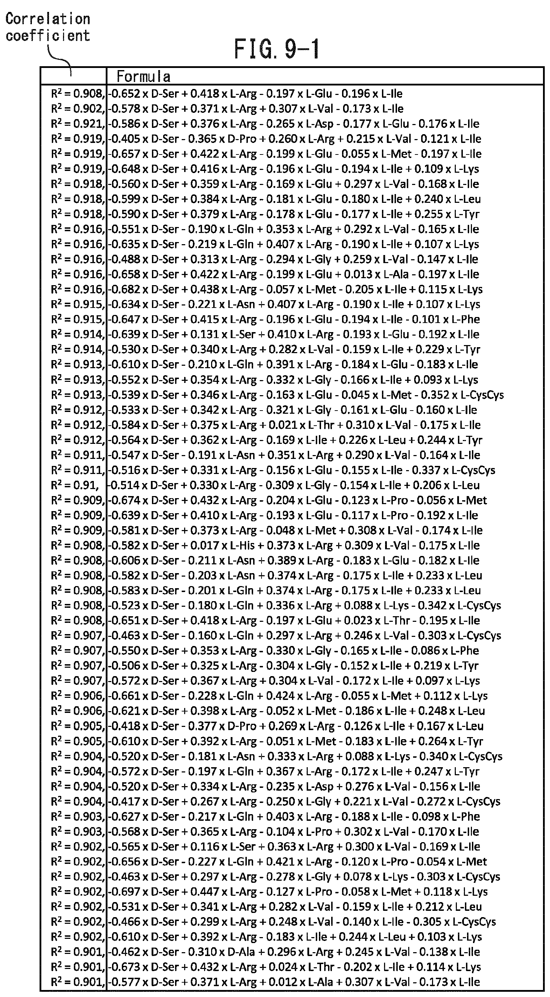

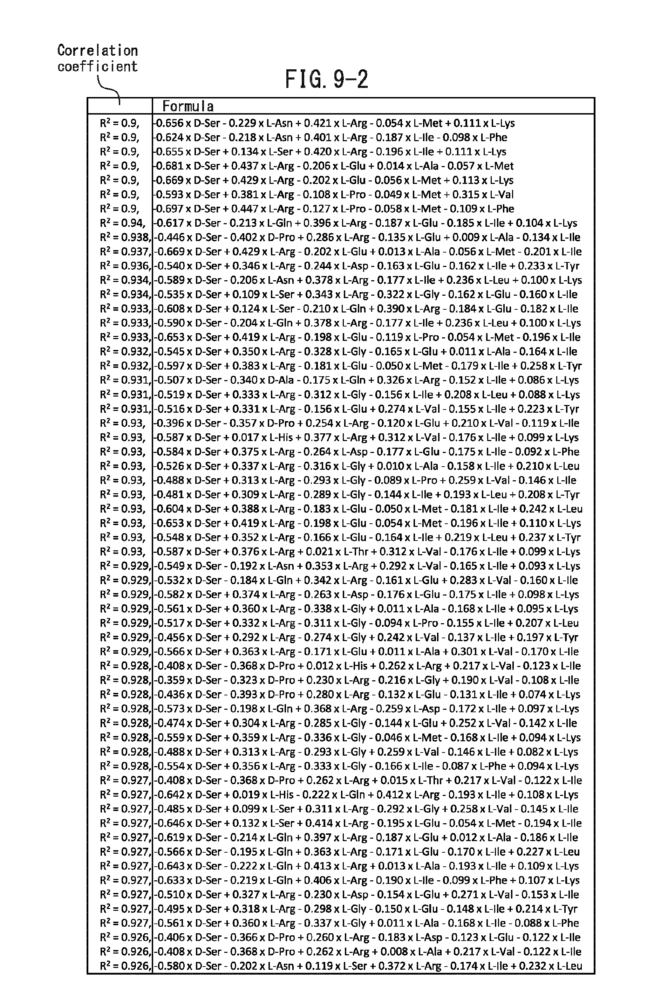

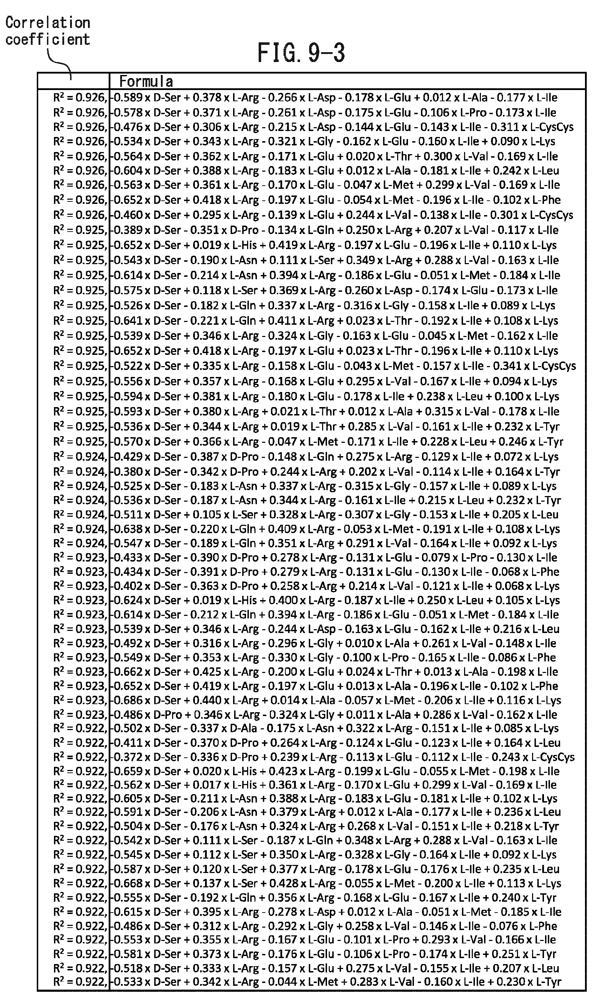

The D- and L-amino acids, creatinine and cystatin C levels in plasma of each subject were used for regression analysis against a dataset of inulin clearance results. The regression analysis was carried out by OPLS using the ropls package of free statistical analysis software "R" (URL: <https://cran.r-project.org/>).
The D- and L-amino acids, creatinine, cystatin C and inulin clearance were converted to logarithms and further standardized. The standardized inulin clearance was used as the response variables and different combinations of the D- and L-amino acids, creatinine and cystatin C were used as the explanatory variables, for all of the methods of OPLS regression analysis. A formula with high correlation coefficient R ($R^2$ value) was extracted (Fig. 4 to Fig. 9).

[Example 2]

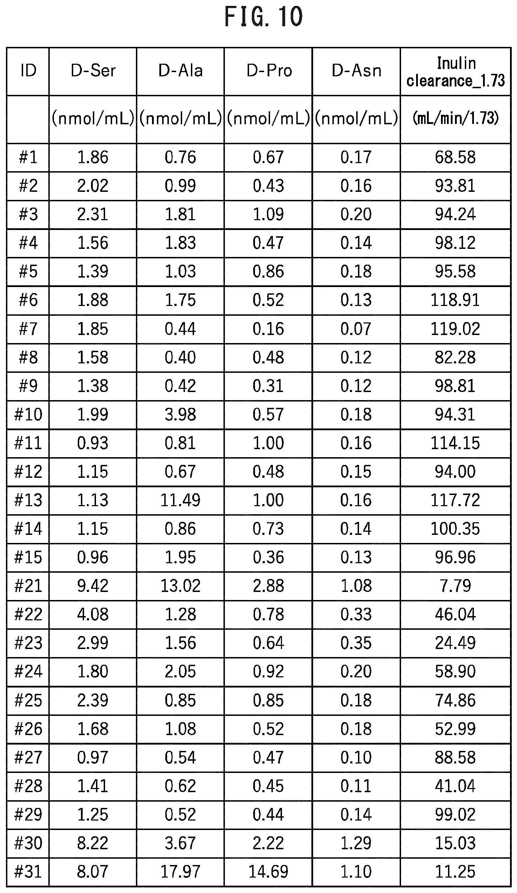

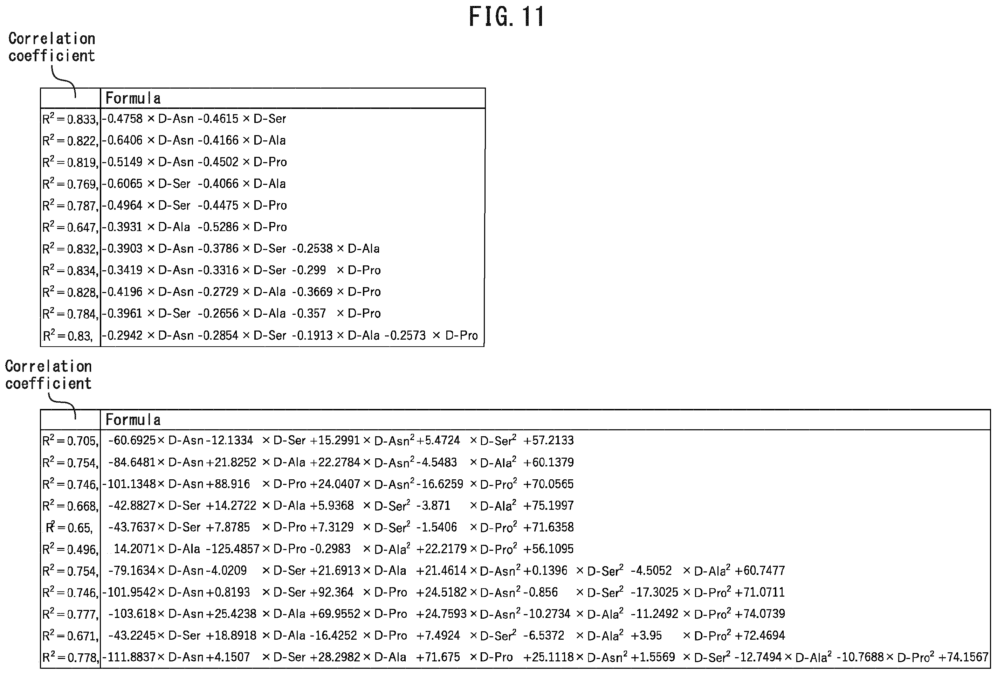

The dataset for D-serine, D-alanine, D-proline and D-asparagine detected at high frequency in blood, and inulin clearance (Fig. 10), obtained with the same specimens and methods as Example 1, was used for regression analysis, and a formula with higher correlation was extracted (Fig. 11). Using the $R^2$ values, the logarithmically transformed kidney function and blood D-amino acid level can be evaluated as having a stronger linear correlation. The first-order and second-order regression analysis results are shown here, but there is no restriction on selection of the nth-order polynomial regression analysis according to the data characteristics.
Formulas derived from combinations of D- and L-amino acids, creatinine and cystatin C which are not shown in Figs. 4 to 9 and 11 are also within the scope of the invention and may be utilized according to the invention.

**Claims**

1.  A method for estimating kidney function of a subject being evaluated, wherein the method includes:

    a step of measuring the levels of D- and L-amino acids in a biological sample from a subject being evaluated;
    a step of estimating the kidney function of the subject being evaluated, based on the value of Y calculated from the levels of D- and L-amino acids in the biological sample from the subject being evaluated, using the following formula (I):

$$Y = a_1 \cdot X_1 + a_2 \cdot X_2 + \ldots + a_n \cdot X_n + b \ (I)$$

    [where

       $a_1$ to $a_n$ represent constants obtained by regression analysis,
       $X_1$ to $X_n$ represent variables for the D- and L-amino acid levels selected by the regression analysis, and
       b represents a constant obtained by the regression analysis],

    wherein formula (I) is predetermined by the regression analysis using D- and L-amino acid levels in biological samples of arbitrary subjects as the explanatory variables and glomerular filtration rates of the arbitrary subjects as the response variables.

2.  The method according to claim 1, wherein the step of estimating the kidney function of the subject being evaluated is a step of estimating the kidney function of the subject being evaluated by estimating the glomerular filtration rate of the subject being evaluated, based on the Y value.

3. The method according to claim 1 or 2, wherein the biological sample is blood, plasma or serum.

4. The method according to any one of claims 1 to 3, wherein formula (I) with a correlation coefficient of $R \geq 0.5$, or with a correlation coefficient of $R \geq 0.8$, is used.

5. The method according to any one of claims 1 to 4, wherein

the explanatory variables further include the level of a factor selected from the group consisting of creatinine and cystatin C, and
$X_1$ to $X_n$ represent variables for the levels of D- and L-amino acids, creatinine or cystatin C selected by the regression analysis.

6. The method according to any one of claims 1 to 4, wherein

the explanatory variables are the standardized values for the logarithm of the D- and L-amino acid levels,
the response variables are the standardized values of the logarithm of the glomerular filtration rates, and
the standardized values for the logarithm of the D- and L-amino acid levels selected by the regression analysis are applied to $X_1$ to $X_n$,
wherein, preferably, the explanatory variables further include the standardized level of the logarithm of the level of a factor selected from the group consisting of creatinine and cystatin C, and
$X_1$ to $X_n$ represent variables for the levels of D- and L-amino acids, creatinine or cystatin C selected by the regression analysis, the standardized levels of the logarithm of the levels of D- and L-amino acids, creatinine or cystatin C selected by the regression analysis being applied to $X_1$ to $X_n$.

7. The method according to any one of claims 1 to 6, wherein $X_1$ to $X_n$ include at least the variable of the level of a factor selected from the group consisting of D-serine, D-alanine, D-proline and D-asparagine.

8. The method according to any one of claims 1 to 7, wherein the glomerular filtration rates of the arbitrary subjects are the glomerular filtration rates calculated by inulin clearance, creatinine clearance, $^{51}$Cr-EDTA clearance, $^{125}$I-sodium iotalamate clearance, $^{99m}$Tc-DTPA clearance, sodium thiosulfate clearance, iohexol clearance, iodixanol clearance or iotalamate clearance.

9. The method according to any one of claims 1 to 7, wherein the subject being evaluated is a subject being evaluated who has been assessed to have suspected kidney disease by a conventional examination method.

10. A system for estimating kidney function of a subject being evaluated, the system including a storage unit, an analytical measurement unit, a data processing unit and an output unit, wherein:

the storage unit stores the following formula (II):

$$Y = a_1 \cdot X_1 + a_2 \cdot X_2 + \ldots + a_n \cdot X_n + b \ (II)$$

[where

$a_1$ to $a_n$ represent constants obtained by regression analysis,
$X_1$ to $X_n$ represent variables for the D- and L-amino acid levels selected by the regression analysis, and
b represents a constant obtained by the regression analysis],

wherein formula (II) is predetermined by the regression analysis using D- and L-amino acid levels in biological samples of arbitrary subjects as the explanatory variables and glomerular filtration rates of the arbitrary subjects as the response variables,
the analytical measurement unit quantifies the levels of D- and L-amino acids in a biological sample from the subject being evaluated,
the data processing unit inputs the D- and L-amino acid levels in the biological sample of the subject being evaluated into formula (II) stored in the storage unit to calculate the value of Y and estimates the kidney function of the subject being evaluated, based on the value of Y, and

the output unit outputs information regarding the estimated kidney function of the subject being evaluated; wherein, preferably, the data processing unit inputs the D- and L-amino acid levels in the biological sample of the subject being evaluated into formula (II) stored in the storage unit to calculate the value of Y, and estimates the glomerular filtration rate of the subject being evaluated, based on the value of Y, thereby estimating the kidney function of the subject being evaluated.

11. The system according to claim 10, wherein the biological sample is blood, plasma or serum.

12. The system according to any one of claims 10 to 11, wherein formula (II) with a correlation coefficient of $R \geq 0.5$ or with a correlation coefficient of $R \geq 0.8$, is used.

13. The system according to any one of claims 10 to 12, wherein the explanatory variables further include the level of a factor selected from the group consisting of creatinine and cystatin C, and

$X_1$ to $X_n$ represent variables for the levels of D- and L-amino acids, creatinine or cystatin C selected by the regression analysis.

14. The system according to any one of claims 10 to 12, wherein the explanatory variables are the standardized values for the logarithm of the D- and L-amino acid levels,

the response variables are the standardized values of the logarithm of the glomerular filtration rates, and
the standardized values for the logarithm of the D- and L-amino acid levels selected by the regression analysis are applied to $X_1$ to $X_n$,
wherein, preferably. the explanatory variables further include the standardized level of the logarithm of the level of a factor selected from the group consisting of creatinine and cystatin C, and
$X_1$ to $X_n$ represent variables for the levels of D- and L-amino acids, creatinine or cystatin C selected by the regression analysis, the standardized levels of the logarithm of the levels of D- and L-amino acids, creatinine or cystatin C selected by the regression analysis being applied to $X_1$ to $X_n$.

15. The system according to any one of claims 10 to 14, wherein $X_1$ to $X_n$ include at least the variable of the level of a factor selected from the group consisting of D-serine, D-alanine, D-proline and D-asparagine.

16. The system according to any one of claims 10 to 15, wherein the glomerular filtration rates of the arbitrary subjects are the glomerular filtration rates calculated by inulin clearance, creatinine clearance, $^{51}$Cr-EDTA clearance, $^{125}$I-sodium iotalamate clearance, $^{99m}$Tc-DTPA clearance, sodium thiosulfate clearance, iohexol clearance, iodixanol clearance or iotalamate clearance.

17. The system according to any one of claims 10 to 16, wherein the subject being evaluated is a subject being evaluated who has been assessed to have suspected kidney disease by a conventional examination method.

## Patentansprüche

1. Verfahren zum Beurteilen der Nierenfunktion eines zu untersuchenden Patienten, wobei das Verfahren umfasst:

einen Schritt zum Messen der Gehalte von D- und L-Aminosäuren in einer biologischen Probe eines zu untersuchenden Patienten;
einen Schritt zum Beurteilen der Nierenfunktion des zu untersuchenden Patienten auf der Grundlage des Wertes von Y, der aus den Gehalten von D- und L-Aminosäuren in der biologischen Probe des zu untersuchenden Patienten unter Verwendung der folgenden Formel (I) berechnet wird:

$$Y = a_1 \cdot X_1 + a_2 \cdot X_2 + \ldots + a_n \cdot X_n + b \qquad (I)$$

[wobei

$a_1$ bis $a_n$ durch Regressionsanalyse ermittelte Konstanten darstellen,
$X_1$ bis $X_n$ Variablen für die D- und L-Aminosäuregehalte darstellen, die durch die Regressionsanalyse ausgewählt wurden, und

b eine durch die Regressionsanalyse erhaltene Konstante darstellt],

wobei die Formel (I) durch die Regressionsanalyse unter Verwendung von D- und L-Aminosäuregehalten in biologischen Proben beliebiger Patienten als erklärende Variablen und der glomerulären Filtrationsraten der beliebigen Patienten als Antwortvariablen vorbestimmt ist.

2. Verfahren nach Anspruch 1, wobei der Schritt des Beurteilens der Nierenfunktion des zu untersuchenden Patienten ein Schritt des Beurteilens der Nierenfunktion des zu untersuchenden Patienten durch Beurteilen der glomerulären Filtrationsrate des zu untersuchenden Patienten auf der Grundlage des Y-Werts ist.

3. Verfahren nach Anspruch 1 oder 2, wobei die biologische Probe Blut, Plasma oder Serum ist.

4. Verfahren nach einem der Ansprüche 1 bis 3, wobei die Formel (I) mit einem Korrelationskoeffizienten von $R \geq 0,5$ oder mit einem Korrelationskoeffizienten von $R \geq 0,8$ verwendet wird.

5. Verfahren nach einem der Ansprüche 1 bis 4, wobei

die erklärenden Variablen ferner den Gehalt eines Faktors umfassen, der ausgewählt ist aus der Gruppe, bestehend aus Kreatinin und Cystatin C, und
$X_1$ bis $X_n$ Variablen für die Gehalte von D- und L-Aminosäuren, Kreatinin oder Cystatin C darstellen, die durch die Regressionsanalyse ausgewählt wurden.

6. Verfahren nach einem der Ansprüche 1 bis 4, wobei

die erklärenden Variablen die standardisierten Werte für den Logarithmus der D- und L-Aminosäuregehalte sind, die Antwortvariablen die standardisierten Werte des Logarithmus der glomerulären Filtrationsraten sind, und die standardisierten Werte für den Logarithmus der D- und L-Aminosäuregehalte, die durch die Regressionsanalyse ausgewählt wurden, auf $X_1$ bis $X_n$ angewendet werden ;
wobei vorzugsweise die erklärenden Variablen ferner den standardisierten Wert des Logarithmus des Gehalts eines Faktors umfassen, der ausgewählt ist aus der Gruppe, bestehend aus Kreatinin und Cystatin C, und $X_1$ bis $X_n$ Variablen für die durch die Regressionsanalyse ausgewählten Gehalte der D- und L-Aminosäuren, des Kreatinins oder des Cystatin C darstellen, wobei die standardisierten Werte des Logarithmus der durch die Regressionsanalyse ausgewählten Gehalte der D- und L-Aminosäuren, des Kreatinins oder des Cystatin C auf $X_1$ bis $X_n$ angewendet werden.

7. Verfahren nach einem der Ansprüche 1 bis 6, wobei $X_1$ bis $X_n$ mindestens die Variable des Gehalts eines Faktors umfassen, der ausgewählt ist aus der Gruppe, bestehend aus D-Serin, D-Alanin, D-Prolin und D-Asparagin.

8. Verfahren nach einem der Ansprüche 1 bis 7, wobei die glomerulären Filtrationsraten der beliebigen Patienten die glomerulären Filtrationsraten sind, die durch Inulinclearance, Kreatininclearance, $^{51}$Cr-EDTA-Clearance, $^{125}$I-Natrium-Iotalamat-Clearance, $^{99m}$Tc-DTPA-Clearance, Natriumthiosulfat-Clearance, Iohexol-Clearance, Iodixanol-Clearance oder Iotalamat-Clearance berechnet werden.

9. Verfahren nach einem der Ansprüche 1 bis 7, wobei der zu untersuchende Patient ein untersuchter Patient ist, bei dem durch ein herkömmliches Untersuchungsverfahren ein Verdacht auf eine Nierenerkrankung festgestellt wurde.

10. System zum Beurteilen der Nierenfunktion eines zu untersuchenden Patienten, wobei das System eine Speichereinheit, eine analytische Messeinheit, eine Datenverarbeitungseinheit und eine Ausgabeeinheit umfasst, wobei:

die Speichereinheit die folgende Formel (II) speichert:

$$Y = a_1 \cdot X_1 + a_2 \cdot X_2 + \ldots + a_n \cdot X_n + b \qquad (II)$$

[wobei

$a_1$ bis $a_n$ durch Regressionsanalyse ermittelte Konstanten darstellen,
$X_1$ bis $X_n$ Variablen für die D- und L-Aminosäuregehalte darstellen, die durch die Regressionsanalyse ausgewählt wurden, und

b eine durch die Regressionsanalyse erhaltene Konstante darstellt],

wobei die Formel (II) durch die Regressionsanalyse unter Verwendung der D- und L-Aminosäuregehalte in biologischen Proben beliebiger Patienten als erklärende Variablen und der glomerulären Filtrationsraten der beliebigen Patienten als Antwortvariablen vorbestimmt ist,

die analytische Messeinheit die Gehalte an D- und L-Aminosäuren in einer biologischen Probe des zu untersuchenden Patienten quantifiziert,

die Datenverarbeitungseinheit die D- und L-Aminosäuregehalte in der biologischen Probe des zu untersuchenden Patienten in die in der Speichereinheit gespeicherte Formel (II) eingibt, um den Wert von Y zu berechnen, und die Nierenfunktion des zu untersuchenden Patienten auf der Grundlage des Wertes von Y beurteilt, und

die Ausgabeeinheit Informationen über die beurteilte Nierenfunktion des zu untersuchenden Patienten ausgibt;

wobei die Datenverarbeitungseinheit vorzugsweise die D- und L-Aminosäuregehalte in der biologischen Probe des zu untersuchenden Patienten in die in der Speichereinheit gespeicherte Formel (II) eingibt, um den Wert von Y zu berechnen, und auf der Grundlage des Wertes von Y die glomeruläre Filtrationsrate des zu untersuchenden Patienten beurteilt, wodurch die Nierenfunktion des zu untersuchenden Patienten beurteilt wird.

11. System nach Anspruch 10, wobei die biologische Probe Blut, Plasma oder Serum ist.

12. System nach einem der Ansprüche 10 bis 11, wobei die Formel (II) mit einem Korrelationskoeffizienten von $R \geq 0{,}5$ oder mit einem Korrelationskoeffizienten von $R \geq 0{,}8$ verwendet wird.

13. System nach einem der Ansprüche 10 bis 12, wobei die erklärenden Variablen ferner den Gehalt eines Faktors umfassen, der ausgewählt ist aus der Gruppe, bestehend aus Kreatinin und Cystatin C, und $X_1$ bis $X_n$ Variablen für die Gehalte von D- und L-Aminosäuren, Kreatinin oder Cystatin C darstellen, die durch die Regressionsanalyse ausgewählt wurden.

14. System nach einem der Ansprüche 10 bis 12, wobei die erklärenden Variablen die standardisierten Werte für den Logarithmus der D- und L-Aminosäuregehalte sind,

die Antwortvariablen die standardisierten Werte des Logarithmus der glomerulären Filtrationsraten sind, und die standardisierten Werte für den Logarithmus der D- und L-Aminosäurehalte, die durch die Regressionsanalyse ausgewählt wurden, auf $X_1$ bis $X_n$ angewendet werden;

wobei vorzugsweise die erklärenden Variablen ferner den standardisierten Wert des Logarithmus des Gehalts eines Faktors umfassen, der ausgewählt ist aus der Gruppe, bestehend aus Kreatinin und Cystatin C, und $X_1$ bis $X_n$ Variablen für die durch die Regressionsanalyse ausgewählten Gehalte der D- und L-Aminosäuren, des Kreatinins oder des Cystatin C darstellen, wobei die standardisierten Werte des Logarithmus der durch die Regressionsanalyse ausgewählten Gehalte der D- und L-Aminosäuren, des Kreatinins oder des Cystatin C auf $X_1$ bis $X_n$ angewendet werden .

15. System nach einem der Ansprüche 10 bis 14, wobei $X_1$ bis $X_n$ mindestens die Variable des Gehalts eines Faktors umfassen, der ausgewählt ist aus der Gruppe, bestehend aus D-Serin, D-Alanin, D-Prolin und D-Asparagin.

16. System nach einem der Ansprüche 10 bis 15, wobei die glomerulären Filtrationsraten der beliebigen Patienten die glomerulären Filtrationsraten sind, die durch Inulinclearance, Kreatininclearance, $^{51}$Cr-EDTA-Clearance, $^{125}$I-Natrium-Iotalamat-Clearance, $^{99m}$Tc-DTPA- - Clearance, Natriumthiosulfat-Clearance, Iohexol-Clearance, Iodixanol-Clearance oder Iotalamat-Clearance berechnet werden.

17. System nach einem der Ansprüche 10 bis 16, wobei der zu untersuchende Patient ein untersuchter Patient ist, bei dem durch ein herkömmliches Untersuchungsverfahren ein Verdacht auf eine Nierenerkrankung festgestellt wurde.

**Revendications**

1. Un procédé d'estimation de la fonction rénale d'un sujet évalué, le procédé comprenant :

une étape de mesure du taux d'acides aminés D et L dans un échantillon biologique provenant d'un sujet évalué ;

une étape d'estimation de la fonction rénale du sujet évalué, sur la base de la valeur Y calculée à partir des taux d'acides aminés D et L dans l'échantillon biologique provenant du sujet évalué, en utilisant la formule suivante (I) :

$$Y = a_1 \cdot X_1 + a_2 \cdot X_2 + \cdot + a_n \cdot X_n + b \ (I)$$

[où
$a_1$ à $a_n$ représentent des constantes obtenues par analyse de régression,
$X_1$ à $X_n$ représentent des variables pour les taux d'acides aminés D et L sélectionnés par l'analyse de régression, et
b représente une constante obtenue par l'analyse de régression],
la formule (I) étant prédéterminée par l'analyse de régression utilisant les taux d'acides aminés D et L dans des échantillons biologiques de sujets arbitraires comme variables explicatives et les taux de filtration glomérulaire des sujets arbitraires comme variables de réponse.

2. Le procédé selon la revendication 1, dans lequel l'étape d'estimation de la fonction rénale du sujet évalué est une étape d'estimation de la fonction rénale du sujet évalué par estimation du taux de filtration glomérulaire du sujet évalué, sur la base de la valeur Y.

3. Le procédé selon la revendication 1 ou 2, dans lequel l'échantillon biologique est du sang, du plasma ou du sérum.

4. Le procédé selon l'une quelconque des revendications 1 à 3, dans lequel la formule (I) ayant un coefficient de corrélation $R \geq 0{,}5$, ou ayant un coefficient de corrélation $R \geq 0{,}8$, est utilisée.

5. Le procédé selon l'une quelconque des revendications 1 à 4, dans lequel

les variables explicatives comprennent en outre le taux d'un facteur sélectionné dans le groupe constitué de la créatinine et de la cystatine C, et
$X_1$ à $X_n$ représentent des variables pour les taux d'acides aminés D et L, de créatinine ou de cystatine C sélectionnés par l'analyse de régression.

6. Le procédé selon l'une quelconque des revendications 1 à 4, dans lequel

les variables explicatives sont les valeurs normalisées pour le logarithme des taux d'acides aminés D et L, les variables de réponse sont les valeurs normalisées du logarithme des taux de filtration glomérulaire, et les valeurs normalisées pour le logarithme des taux d'acides aminés D et L sélectionnées par l'analyse de régression sont appliquées à $X_1$ à $X_n$ ;
dans lequel, de préférence, les variables explicatives comprennent en outre le taux normalisé du logarithme du taux d'un facteur sélectionné dans le groupe constitué de la créatinine et de la cystatine C, et
$X_1$ à $X_n$ représentent des variables pour les taux d'acides aminés D et L, de créatinine ou de cystatine C sélectionnés par l'analyse de régression, les taux normalisés du logarithme des taux d'acides aminés D et L, de créatinine ou de cystatine C sélectionnés par l'analyse de régression étant appliqués à $X_1$ à $X_n$.

7. Le procédé selon l'une quelconque des revendications 1 à 6, dans lequel $X_1$ à $X_n$ comprennent au moins la variable du taux d'un facteur sélectionné dans le groupe constitué de la D-sérine, de la D-alanine, de la D-proline et de la D-asparagine.

8. Le procédé selon l'une quelconque des revendications 1 à 7, dans lequel les taux de filtration glomérulaire des sujets arbitraires sont les taux de filtration glomérulaire calculés par clairance à l'inuline, clairance à la créatinine, clairance du $^{51}$Cr-EDTA, clairance du $^{125}$I-iodotalamate de sodium, clairance du $^{99}$mTc-DTPA, clairance au thiosulfate de sodium, clairance à l'iohexol, clairance à l'iodixanol ou clairance à l'iodotalamate.

9. Le procédé selon l'une quelconque des revendications 1 à 7, dans lequel le sujet évalué est un sujet évalué qui a été évalué comme présentant une maladie rénale suspectée par une méthode d'examen conventionnelle.

10. Un système d'estimation de la fonction rénale d'un sujet évalué, le système comprenant une unité de stockage, une unité de mesure analytique, une unité de traitement de données et une unité de sortie, dans lequel :

l'unité de stockage stocke la formule suivante (II) :

$$Y = a_1 \cdot X_1 + a_2 . X_2 + \ldots + a_n . X_n + b \ (II)$$

[où

$a_1$ à $a_n$ représentent des constantes obtenues par analyse de régression,

$X_1$ à $X_n$ représentent des variables pour les taux d'acides aminés D et L sélectionnés par l'analyse de régression, et

b représente une constante obtenue par l'analyse de régression],

la formule (II) étant prédéterminée par l'analyse de régression utilisant les taux d'acides aminés D et L dans des échantillons biologiques de sujets arbitraires comme variables explicatives et les taux de filtration glomérulaire des sujets arbitraires comme variables de réponse,

l'unité de mesure analytique quantifie les taux d'acides aminés D et L dans un échantillon biologique provenant du sujet évalué,

l'unité de traitement de données saisit les taux d'acides aminés D et L dans l'échantillon biologique du sujet évalué dans la formule (II) stockée dans l'unité de stockage pour calculer la valeur de Y et estime la fonction rénale du sujet évalué, sur la base de la valeur de Y, et

l'unité de sortie délivre une information concernant la fonction rénale estimée du sujet évalué ;

dans lequel, de préférence, l'unité de traitement de données saisit les taux d'acides aminés D et L dans l'échantillon biologique du sujet évalué dans la formule (II) stockée dans l'unité de stockage pour calculer la valeur de Y, et estime le taux de filtration glomérulaire du sujet évalué, sur la base de la valeur de Y, permettant ainsi d'estimer la fonction rénale du sujet évalué.

11. Le système selon la revendication 10, dans lequel l'échantillon biologique est du sang, du plasma ou du sérum.

12. Le système selon l'une quelconque des revendications 10 à 11, dans lequel la formule (II) ayant un coefficient de corrélation $R \geq 0{,}5$ ou ayant un coefficient de corrélation $R \geq 0{,}8$, est utilisée.

13. Le système selon l'une quelconque des revendications 10 à 12, dans lequel les variables explicatives comprennent en outre le taux d'un facteur sélectionné dans le groupe constitué de la créatinine et de la cystatine C, et

$X_1$ à $X_n$ représentent des variables pour les taux d'acides aminés D et L, de créatinine ou de cystatine C sélectionnés par l'analyse de régression.

14. Le système selon l'une quelconque des revendications 10 à 12, dans lequel les variables explicatives sont les valeurs normalisées pour le logarithme des taux d'acides aminés D et L,

les variables de réponse sont les valeurs normalisées du logarithme des taux de filtration glomérulaire, et

les valeurs normalisées pour le logarithme des taux d'acides aminés D et L sélectionnées par l'analyse de régression sont appliquées à $X_1$ à $X_n$ ;

dans lequel, de préférence, les variables explicatives comprennent en outre le taux normalisé du logarithme du taux d'un facteur sélectionné dans le groupe constitué de la créatinine et de la cystatine C, et

$X_1$ à $X_n$ représentent des variables pour les taux d'acides aminés D et L, de créatinine ou de cystatine C sélectionnés par l'analyse de régression, les taux normalisés du logarithme des taux d'acides aminés D et L, de créatinine ou de cystatine C sélectionnés par l'analyse de régression étant appliqués à $X_1$ à $X_n$.

15. Le système selon l'une quelconque des revendications 10 à 14, dans lequel $X_1$ à $X_n$ comprennent au moins la variable du taux d'un facteur sélectionné dans le groupe constitué de la D-sérine, de la D-alanine, de la D-proline et de la D-asparagine.

16. Le système selon l'une quelconque des revendications 10 à 15, dans lequel les taux de filtration glomérulaire des sujets arbitraires sont les taux de filtration glomérulaire calculés par clairance à l'inuline, clairance à la créatinine, clairance du ${}^{51}$Cr-EDTA, clairance du ${}^{125}$I-iodotalamate de sodium, clairance du ${}^{99m}$Tc-DTPA, clairance au thio-sulfate de sodium, la clairance à l'iohexol, ou clairance à l'iodotalamate.

17. Le système selon l'une quelconque des revendications 10 à 16, dans lequel le sujet évalué est un sujet évalué qui a été évalué comme présentant une maladie rénale suspectée par une méthode d'examen conventionnelle.

FIG.1

10

11 Storage unit

12 Input unit

13 Analytical measurement unit

14 Data processing unit

15 Output unit

FIG. 2

```
                    ┌─────────┐
                    │  START  │
                    └─────────┘
                         │
                         ▼
┌───────────────────────────────────────────────────────────────────────┐
│  Storage in storage unit of  threshold for evaluating kidney function   │
│  inputted through input device; and  formula predetemined by regression │
│  analysis using D-and L-amino acid levels in biological samples of       │
│  arbitrary subjects as explanatory variables, and glomerular filtration  │
│  rates of arbitrary subject as response variables.                       │
└───────────────────────────────────────────────────────────────────────┘
                         │
                         ▼
┌───────────────────────────────────────────────────────────────────────┐
│  Storage in storage unit of D-and L-amino acid levels in biological      │
│  sample, inputted through input device or quantified by analytical       │
│  measurement unit.                                                       │
└───────────────────────────────────────────────────────────────────────┘
                         │
                         ▼
┌───────────────────────────────────────────────────────────────────────┐
│  Readout of factors including D-and L-amino acid levels in biological    │
│  sample stored in storage unit and calculation formula stored in storage │
│  unit, calculation of clinical diagnosis value in data processing unit,  │
│  and storage in storage unit.                                            │
└───────────────────────────────────────────────────────────────────────┘
                         │
                         ▼
┌───────────────────────────────────────────────────────────────────────┐
│  Readout of stored clinical diagnosis value and threshold for evaluating │
│  kidney function, comparison of clinical diagnosis value and threshold   │
│  by data processing unit to evaluate kidney function, and output of       │
│  evaluation results to output unit.                                      │
└───────────────────────────────────────────────────────────────────────┘
                         │
                         ▼
                    ┌─────────┐
                    │   END   │
                    └─────────┘
```

FIG. 3-1

| ID | D-Ser | D-Ala | D-Pro | L-His | L-Asn | L-Ser | L-Gln | L-Arg | L-Asp | L-Gly | L-Glu | L-Thr | L-Ala |
|---|---|---|---|---|---|---|---|---|---|---|---|---|---|
| | (nmol/mL) | (nmol/mL) | (nmol/mL) | (nmol/mL) | (nmol/mL) | (nmol/mL) | (nmol/mL) | (nmol/mL) | (nmol/mL) | (nmol/mL) | (nmol/mL) | (nmol/mL) | (nmol/mL) |
| #1 | 1.86 | 0.76 | 0.67 | 89.76 | 44.40 | 90.07 | 571.47 | 66.66 | 3.39 | 240.90 | 41.78 | 139.65 | 468.09 |
| #2 | 2.02 | 0.99 | 0.43 | 50.35 | 43.68 | 107.63 | 591.84 | 62.85 | 2.48 | 177.55 | 27.08 | 117.65 | 444.74 |
| #3 | 2.31 | 1.81 | 1.09 | 85.94 | 41.08 | 121.78 | 524.76 | 76.06 | 1.50 | 233.65 | 17.56 | 118.87 | 256.50 |
| #4 | 1.56 | 1.83 | 0.47 | 75.05 | 34.55 | 91.18 | 434.85 | 64.65 | 1.65 | 183.28 | 31.91 | 111.92 | 455.54 |
| #5 | 1.39 | 1.03 | 0.86 | 60.06 | 40.43 | 93.35 | 498.17 | 82.95 | 4.19 | 199.96 | 28.85 | 118.01 | 342.00 |
| #6 | 1.88 | 1.75 | 0.52 | 57.87 | 41.41 | 114.08 | 553.19 | 55.28 | 5.90 | 241.70 | 79.89 | 110.99 | 393.96 |
| #7 | 1.85 | 0.44 | 0.16 | 55.32 | 30.04 | 87.55 | 332.82 | 44.23 | 1.77 | 120.08 | 19.77 | 72.50 | 286.66 |
| #8 | 1.58 | 0.40 | 0.48 | 82.05 | 38.87 | 114.83 | 594.61 | 57.56 | 3.03 | 196.49 | 19.87 | 147.06 | 217.16 |
| #9 | 1.38 | 0.42 | 0.31 | 64.27 | 33.02 | 111.16 | 514.80 | 51.55 | 1.56 | 214.84 | 14.93 | 108.53 | 231.68 |
| #10 | 1.99 | 3.98 | 0.57 | 88.80 | 41.42 | 86.35 | 583.50 | 84.60 | 3.90 | 184.03 | 41.53 | 108.47 | 286.00 |
| #11 | 0.93 | 0.81 | 1.00 | 63.63 | 33.08 | 78.58 | 422.80 | 54.05 | 4.40 | 146.40 | 66.63 | 79.68 | 269.96 |
| #12 | 1.15 | 0.67 | 0.48 | 66.87 | 50.59 | 93.65 | 497.37 | 71.25 | 2.01 | 173.36 | 22.11 | 101.24 | 254.76 |
| #13 | 1.13 | 11.49 | 1.00 | 61.23 | 39.80 | 114.31 | 428.63 | 47.93 | 2.07 | 167.89 | 25.13 | 82.87 | 222.48 |
| #14 | 1.15 | 0.86 | 0.73 | 56.75 | 37.35 | 89.10 | 421.75 | 53.15 | 2.64 | 166.60 | 47.84 | 85.34 | 324.42 |
| #15 | 0.96 | 1.95 | 0.36 | 51.84 | 37.69 | 96.44 | 482.85 | 66.10 | 1.91 | 197.44 | 23.36 | 87.75 | 326.64 |
| #21 | 9.42 | 13.02 | 2.88 | 40.02 | 52.57 | 106.94 | 677.56 | 58.62 | 3.50 | 345.06 | 32.99 | 90.27 | 326.13 |
| #22 | 4.08 | 1.28 | 0.78 | 103.00 | 65.07 | 130.47 | 716.00 | 60.76 | 3.78 | 375.04 | 80.04 | 155.65 | 495.27 |
| #23 | 2.99 | 1.56 | 0.64 | 95.87 | 60.55 | 110.89 | 616.60 | 24.90 | 2.75 | 390.26 | 26.69 | 168.91 | 340.89 |
| #24 | 1.80 | 2.05 | 0.92 | 81.39 | 51.26 | 105.07 | 418.95 | 26.71 | 3.98 | 210.72 | 40.46 | 125.62 | 464.70 |
| #25 | 2.39 | 0.85 | 0.85 | 74.34 | 47.81 | 122.03 | 430.99 | 25.54 | 4.81 | 163.67 | 79.69 | 138.90 | 328.50 |
| #26 | 1.68 | 1.08 | 0.52 | 109.59 | 52.19 | 114.65 | 508.04 | 27.92 | 5.13 | 219.24 | 42.69 | 125.70 | 292.54 |
| #27 | 0.97 | 0.54 | 0.47 | 86.15 | 46.79 | 123.18 | 496.68 | 26.01 | 2.94 | 187.89 | 36.36 | 132.82 | 449.71 |
| #28 | 1.41 | 0.62 | 0.45 | 84.14 | 46.60 | 129.06 | 455.82 | 17.16 | 4.78 | 173.51 | 49.79 | 120.26 | 247.64 |
| #29 | 1.25 | 0.52 | 0.44 | 97.82 | 54.67 | 124.07 | 482.90 | 35.90 | 4.24 | 213.41 | 44.44 | 85.27 | 511.70 |
| #30 | 8.22 | 3.67 | 2.22 | 62.78 | 31.89 | 72.94 | 409.80 | 16.64 | 5.92 | 176.11 | 83.32 | 46.79 | 257.23 |
| #31 | 8.07 | 17.97 | 14.69 | 79.55 | 42.01 | 75.52 | 499.55 | 10.91 | 4.76 | 227.32 | 48.87 | 123.01 | 326.36 |

FIG. 3-2

| ID | L-Pro | L-Met | L-Val | L-Ile | L-Leu | L-Phe | L-Lys | L-CysCys | L-Tyr | Cystatin C | Creatinine | Inulin clearance 1.73 |
|---|---|---|---|---|---|---|---|---|---|---|---|---|
| | (nmol/mL) | (nmol/mL) | (nmol/mL) | (nmol/mL) | (nmol/mL) | (nmol/mL) | (nmol/mL) | (nmol/mL) | (nmol/mL) | (mg/dL) | (mg/dL) | (mL/min/1.73) |
| #1 | 197.12 | 35.28 | 251.24 | 79.15 | 140.23 | 57.82 | 143.76 | 29.31 | 65.70 | 0.86 | 0.95 | 68.58 |
| #2 | 177.15 | 16.00 | 258.05 | 72.45 | 143.13 | 57.88 | 188.91 | 25.69 | 64.88 | 0.82 | 0.75 | 93.81 |
| #3 | 123.23 | 10.25 | 221.86 | 47.23 | 111.83 | 49.11 | 130.62 | 28.48 | 43.42 | 0.91 | 0.93 | 94.24 |
| #4 | 133.10 | 16.39 | 311.43 | 74.12 | 142.80 | 58.20 | 156.36 | 35.63 | 73.45 | 0.63 | 0.73 | 98.12 |
| #5 | 182.09 | 35.28 | 207.12 | 58.49 | 107.25 | 47.76 | 149.54 | 25.40 | 58.81 | 0.78 | 0.89 | 95.58 |
| #6 | 179.77 | 18.92 | 266.97 | 75.74 | 142.99 | 82.91 | 172.90 | 27.53 | 84.58 | 0.80 | 0.84 | 118.91 |
| #7 | 104.95 | 10.95 | 162.07 | 42.18 | 94.00 | 54.94 | 142.58 | 20.92 | 67.72 | 0.55 | 0.54 | 119.02 |
| #8 | 140.96 | 22.69 | 190.39 | 48.02 | 95.10 | 45.37 | 136.75 | 25.37 | 50.27 | 0.72 | 0.67 | 82.28 |
| #9 | 105.25 | 21.54 | 160.49 | 43.21 | 80.48 | 39.06 | 129.27 | 21.07 | 46.88 | 0.74 | 0.50 | 98.81 |
| #10 | 123.33 | 21.64 | 263.79 | 72.41 | 144.64 | 66.96 | 168.33 | 30.91 | 89.05 | 0.86 | 0.82 | 94.31 |
| #11 | 114.79 | 23.58 | 233.21 | 66.45 | 136.04 | 54.80 | 138.77 | 40.35 | 51.14 | 0.91 | 0.71 | 114.15 |
| #12 | 99.94 | 21.97 | 246.56 | 66.73 | 140.59 | 52.59 | 135.76 | 38.77 | 48.15 | 0.63 | 0.64 | 94.00 |
| #13 | 90.82 | 23.96 | 213.35 | 64.64 | 132.37 | 50.08 | 124.68 | 32.00 | 49.80 | 0.65 | 0.68 | 117.72 |
| #14 | 89.12 | 23.15 | 230.64 | 69.15 | 132.29 | 49.94 | 152.26 | 37.45 | 52.08 | 0.78 | 0.81 | 100.35 |
| #15 | 119.72 | 25.90 | 191.89 | 62.67 | 117.06 | 46.91 | 137.01 | 29.99 | 51.15 | 0.82 | 0.80 | 96.96 |
| #21 | 141.47 | 26.93 | 145.97 | 130.24 | 90.34 | 53.45 | 134.92 | 61.50 | 58.15 | 2.70 | 3.34 | 7.79 |
| #22 | 217.69 | 34.52 | 252.05 | 89.66 | 155.15 | 71.51 | 234.08 | 63.90 | 82.96 | 1.48 | 1.84 | 46.04 |
| #23 | 146.30 | 27.80 | 205.92 | 59.65 | 126.42 | 62.73 | 151.10 | 28.94 | 44.43 | 1.52 | 1.58 | 24.49 |
| #24 | 138.84 | 25.60 | 267.61 | 63.50 | 130.58 | 66.45 | 162.28 | 45.02 | 70.28 | 1.14 | 0.83 | 58.90 |
| #25 | 143.12 | 23.39 | 264.75 | 62.15 | 139.26 | 64.74 | 192.54 | 39.10 | 59.91 | 0.90 | 1.14 | 74.86 |
| #26 | 117.96 | 22.87 | 256.97 | 60.93 | 139.48 | 65.66 | 187.97 | 30.53 | 54.42 | 0.91 | 0.75 | 52.99 |
| #27 | 228.54 | 27.60 | 211.81 | 59.02 | 100.67 | 45.39 | 174.29 | 28.94 | 56.41 | 0.67 | 0.57 | 88.58 |
| #28 | 123.48 | 22.21 | 230.36 | 52.79 | 122.02 | 73.76 | 179.88 | 32.94 | 72.90 | 0.67 | 0.43 | 41.04 |
| #29 | 287.57 | 42.64 | 225.43 | 61.85 | 132.60 | 55.51 | 203.81 | 29.69 | 67.07 | 0.83 | 0.74 | 99.02 |
| #30 | 184.22 | 15.78 | 192.72 | 65.41 | 103.54 | 58.63 | 143.58 | 86.22 | 41.51 | 3.54 | 3.60 | 15.03 |
| #31 | 141.16 | 19.67 | 156.82 | 44.30 | 92.32 | 51.38 | 118.51 | 31.72 | 30.75 | 3.36 | 5.71 | 11.25 |

## FIG. 4

Regression results by regression formula with highest $R^2$ value($R^2$=0.979)

-0.36053 x D-Pro + 0.081811 x L-Ser + 0.256673 x L-Arg -0.24043 x L-Gly - 0.12101 x L-Glu + 0.014289 x L-Thr + 0.007967 x L-Ala -0.07297 x L-Pro - 0.0332 x L-Met + 0.212387 x L-Val - 0.11997 x L-Ile -0.06269 x L-Phe + 0.067539 x L-Lys - 0.26144 x L-CysCys + 0.172829 x L-Tyr

EP 4 083 628 B1

# FIG. 5

EP 4 083 628 B1

Regression results by regression formula with highest $R^2$ value, including frequent appearing D-Ser, L-Arg, L-Ile ($R^2$=0.976)

-0.329971942 x D-Ser -0.297467841 x D-Pro + 0.067501793 x L-Ser +0.211779129 x L-Arg −
0.149124985 x L-Asp -0.198379333 x L-Gly -0.099845616 x L-Glu + 0.006573161 x L-Ala −
0.060208696 x L-Pro + 0.175238479 x L-Val -0.098984946 x L-Ile -0.05172525 x L-Phe +
0.05572616 x L-Lys

# FIG. 6

Using 2 amino acids, $R^2 \geq 0.8$

| Correlation coefficient | Formula |
|---|---|
| $R^2$ = 0.827, | -0.67 x D-Ser + 0.43 x L-Arg |
| $R^2$ = 0.807, | -0.917 x D-Ser - 0.076 x L-Met |

Using 3 amino acids, $R^2 \geq 0.85$

| Correlation coefficient | Formula |
|---|---|
| $R^2$ = 0.864, | -0.696 x D-Ser + 0.447 x L-Arg - 0.058 x L-Met |
| $R^2$ = 0.855, | -0.651 x D-Ser + 0.418 x L-Arg - 0.195 x L-Ile |
| $R^2$ = 0.855, | -0.551 x D-Ser + 0.354 x L-Arg - 0.331 x L-Gly |
| $R^2$ = 0.851, | -0.816 x D-Ser - 0.068 x L-Met + 0.352 x L-Tyr |
| $R^2$ = 0.851, | -0.636 x D-Ser - 0.219 x L-Gln + 0.408 x L-Arg |

FIG. 7　Using 2 amino acids + creatinine and/or cystatin C, $R^2 \geq 0.8$

| Correlation coefficient | Formula |
|---|---|
| $R^2 = 0.863,$ | -0.398 x D-Ser + 0.256 x L-Arg - 0.412 x CystatinC |
| $R^2 = 0.838,$ | -0.407 x D-Ser + 0.261 x L-Arg - 0.395 x Creatinine |
| $R^2 = 0.853,$ | -0.285 x D-Ser + 0.183 x L-Arg - 0.294 x CystatinC - 0.277 x Creatinine |
| $R^2 = 0.847,$ | -0.467 x D-Ser - 0.039 x L-Met - 0.483 x CystatinC |
| $R^2 = 0.796,$ | -0.467 x D-Ser - 0.039 x L-Met - 0.453 x Creatinine |
| $R^2 = 0.824,$ | -0.312 x D-Ser - 0.026 x L-Met - 0.323 x CystatinC - 0.303 x Creatinine |

Using 3 amino acids + creatinine and/or cystatin C, $R^2 \geq 0.85$

| Correlation coefficient | Formula |
|---|---|
| $R^2 = 0.878,$ | -0.404 x D-Ser + 0.259 x L-Arg - 0.034 x L-Met - 0.417 x CystatinC |
| $R^2 = 0.852,$ | -0.412 x D-Ser + 0.265 x L-Arg - 0.034 x L-Met - 0.400 x Creatinine |
| $R^2 = 0.861,$ | -0.287 x D-Ser + 0.184 x L-Arg - 0.024 x L-Met - 0.297 x CystatinC - 0.279 x Creatinine |
| $R^2 = 0.878,$ | -0.391 x D-Ser + 0.251 x L-Arg - 0.117 x L-Ile - 0.404 x CystatinC |
| $R^2 = 0.855,$ | -0.400 x D-Ser + 0.257 x L-Arg - 0.120 x L-Ile - 0.388 x Creatinine |
| $R^2 = 0.865,$ | -0.282 x D-Ser + 0.181 x L-Arg - 0.084 x L-Ile - 0.291 x CystatinC - 0.274 x Creatinine |
| $R^2 = 0.88,$ | -0.354 x D-Ser + 0.227 x L-Arg - 0.213 x L-Gly - 0.366 x CystatinC |
| $R^2 = 0.86,$ | -0.362 x D-Ser + 0.232 x L-Arg - 0.217 x L-Gly - 0.351 x Creatinine |
| $R^2 = 0.87,$ | -0.263 x D-Ser + 0.169 x L-Arg - 0.158 x L-Gly - 0.272 x CystatinC - 0.255 x Creatinine |
| $R^2 = 0.856,$ | -0.433 x D-Ser - 0.036 x L-Met + 0.187 x L-Tyr - 0.448 x CystatinC |
| $R^2 = 0.812,$ | -0.434 x D-Ser - 0.036 x L-Met + 0.188 x L-Tyr - 0.422 x Creatinine |
| $R^2 = 0.83,$ | -0.296 x D-Ser - 0.025 x L-Met + 0.128 x L-Tyr - 0.306 x CystatinC - 0.288 x Creatinine |
| $R^2 = 0.88,$ | -0.387 x D-Ser - 0.134 x L-Gln + 0.249 x L-Arg - 0.400 x CystatinC |
| $R^2 = 0.857,$ | -0.396 x D-Ser - 0.137 x L-Gln + 0.254 x L-Arg - 0.385 x Creatinine |
| $R^2 = 0.87,$ | -0.281 x D-Ser - 0.097 x L-Gln + 0.180 x L-Arg - 0.290 x CystatinC - 0.273 x Creatinine |

EP 4 083 628 B1

FIG. 8

Using D-serine + creatinine and/or cystatin C

| Correlation coefficient | Formula |
|---|---|
| $R^2 = 0.836,$ | -0.462461746 x D-Ser - 0.478007012 x CystatinC |
| $R^2 = 0.782,$ | -0.459970324 x D-Ser - 0.446929105 x Creatinine |
| $R^2 = 0.819,$ | -0.481080775 x CystatinC - 0.452239376 x Creatinine |
| $R^2 = 0.847,$ | -0.3215076 x D-Ser - 0.33231481 x CystatinC - 0.312392118 x Creatinine |

EP 4 083 628 B1

Correlation
coefficient

# FIG. 9-1

| | Formula |
|---|---|
| R² = 0.908, | -0.652 x D-Ser + 0.418 x L-Arg - 0.197 x L-Glu - 0.196 x L-Ile |
| R² = 0.902, | -0.578 x D-Ser + 0.371 x L-Arg + 0.307 x L-Val - 0.173 x L-Ile |
| R² = 0.921, | -0.586 x D-Ser + 0.376 x L-Arg - 0.265 x L-Asp - 0.177 x L-Glu - 0.176 x L-Ile |
| R² = 0.919, | -0.405 x D-Ser - 0.365 x D-Pro + 0.260 x L-Arg + 0.215 x L-Val - 0.121 x L-Ile |
| R² = 0.919, | -0.657 x D-Ser + 0.422 x L-Arg - 0.199 x L-Glu - 0.055 x L-Met - 0.197 x L-Ile |
| R² = 0.919, | -0.648 x D-Ser + 0.416 x L-Arg - 0.196 x L-Glu - 0.194 x L-Ile + 0.109 x L-Lys |
| R² = 0.918, | -0.560 x D-Ser + 0.359 x L-Arg - 0.169 x L-Glu + 0.297 x L-Val - 0.168 x L-Ile |
| R² = 0.918, | -0.599 x D-Ser + 0.384 x L-Arg - 0.181 x L-Glu - 0.180 x L-Ile + 0.240 x L-Leu |
| R² = 0.918, | -0.590 x D-Ser + 0.379 x L-Arg - 0.178 x L-Glu - 0.177 x L-Ile + 0.255 x L-Tyr |
| R² = 0.916, | -0.551 x D-Ser - 0.190 x L-Gln + 0.353 x L-Arg + 0.292 x L-Val - 0.165 x L-Ile |
| R² = 0.916, | -0.635 x D-Ser - 0.219 x L-Gln + 0.407 x L-Arg - 0.190 x L-Ile + 0.107 x L-Lys |
| R² = 0.916, | -0.488 x D-Ser + 0.313 x L-Arg - 0.294 x L-Gly + 0.259 x L-Val - 0.147 x L-Ile |
| R² = 0.916, | -0.658 x D-Ser + 0.422 x L-Arg - 0.199 x L-Glu + 0.013 x L-Ala - 0.197 x L-Ile |
| R² = 0.916, | -0.682 x D-Ser + 0.438 x L-Arg - 0.057 x L-Met - 0.205 x L-Ile + 0.115 x L-Lys |
| R² = 0.915, | -0.634 x D-Ser - 0.221 x L-Asn + 0.407 x L-Arg - 0.190 x L-Ile + 0.107 x L-Lys |
| R² = 0.915, | -0.647 x D-Ser + 0.415 x L-Arg - 0.196 x L-Glu - 0.194 x L-Ile - 0.101 x L-Phe |
| R² = 0.914, | -0.639 x D-Ser + 0.131 x L-Ser + 0.410 x L-Arg - 0.193 x L-Glu - 0.192 x L-Ile |
| R² = 0.914, | -0.530 x D-Ser + 0.340 x L-Arg + 0.282 x L-Val - 0.159 x L-Ile + 0.229 x L-Tyr |
| R² = 0.913, | -0.610 x D-Ser - 0.210 x L-Gln + 0.391 x L-Arg - 0.184 x L-Glu - 0.183 x L-Ile |
| R² = 0.913, | -0.552 x D-Ser + 0.354 x L-Arg - 0.332 x L-Gly - 0.166 x L-Ile + 0.093 x L-Lys |
| R² = 0.913, | -0.539 x D-Ser + 0.346 x L-Arg - 0.163 x L-Glu - 0.045 x L-Met - 0.352 x L-CysCys |
| R² = 0.912, | -0.533 x D-Ser + 0.342 x L-Arg - 0.321 x L-Gly - 0.161 x L-Glu - 0.160 x L-Ile |
| R² = 0.912, | -0.584 x D-Ser + 0.375 x L-Arg + 0.021 x L-Thr + 0.310 x L-Val - 0.175 x L-Ile |
| R² = 0.912, | -0.564 x D-Ser + 0.362 x L-Arg - 0.169 x L-Ile + 0.226 x L-Leu + 0.244 x L-Tyr |
| R² = 0.911, | -0.547 x D-Ser - 0.191 x L-Asn + 0.351 x L-Arg + 0.290 x L-Val - 0.164 x L-Ile |
| R² = 0.911, | -0.516 x D-Ser + 0.331 x L-Arg - 0.156 x L-Glu - 0.155 x L-Ile - 0.337 x L-CysCys |
| R² = 0.91, | -0.514 x D-Ser + 0.330 x L-Arg - 0.309 x L-Gly - 0.154 x L-Ile + 0.206 x L-Leu |
| R² = 0.909, | -0.674 x D-Ser + 0.432 x L-Arg - 0.204 x L-Glu - 0.123 x L-Pro - 0.056 x L-Met |
| R² = 0.909, | -0.639 x D-Ser + 0.410 x L-Arg - 0.193 x L-Glu - 0.117 x L-Pro - 0.192 x L-Ile |
| R² = 0.909, | -0.581 x D-Ser + 0.373 x L-Arg - 0.048 x L-Met + 0.308 x L-Val - 0.174 x L-Ile |
| R² = 0.908, | -0.582 x D-Ser + 0.017 x L-His + 0.373 x L-Arg + 0.309 x L-Val - 0.175 x L-Ile |
| R² = 0.908, | -0.606 x D-Ser - 0.211 x L-Asn + 0.389 x L-Arg - 0.183 x L-Glu - 0.182 x L-Ile |
| R² = 0.908, | -0.582 x D-Ser - 0.203 x L-Asn + 0.374 x L-Arg - 0.175 x L-Ile + 0.233 x L-Leu |
| R² = 0.908, | -0.583 x D-Ser - 0.201 x L-Gln + 0.374 x L-Arg - 0.175 x L-Ile + 0.233 x L-Leu |
| R² = 0.908, | -0.523 x D-Ser - 0.180 x L-Gln + 0.336 x L-Arg + 0.088 x L-Lys - 0.342 x L-CysCys |
| R² = 0.908, | -0.651 x D-Ser + 0.418 x L-Arg - 0.197 x L-Glu + 0.023 x L-Thr - 0.195 x L-Ile |
| R² = 0.907, | -0.463 x D-Ser - 0.160 x L-Gln + 0.297 x L-Arg + 0.246 x L-Val - 0.303 x L-CysCys |
| R² = 0.907, | -0.550 x D-Ser + 0.353 x L-Arg - 0.330 x L-Gly - 0.165 x L-Ile - 0.086 x L-Phe |
| R² = 0.907, | -0.506 x D-Ser + 0.325 x L-Arg - 0.304 x L-Gly - 0.152 x L-Ile + 0.219 x L-Tyr |
| R² = 0.907, | -0.572 x D-Ser + 0.367 x L-Arg + 0.304 x L-Val - 0.172 x L-Ile + 0.097 x L-Lys |
| R² = 0.906, | -0.661 x D-Ser - 0.228 x L-Gln + 0.424 x L-Arg - 0.055 x L-Met + 0.112 x L-Lys |
| R² = 0.906, | -0.621 x D-Ser + 0.398 x L-Arg - 0.052 x L-Met - 0.186 x L-Ile + 0.248 x L-Leu |
| R² = 0.905, | -0.418 x D-Ser - 0.377 x D-Pro + 0.269 x L-Arg - 0.126 x L-Ile + 0.167 x L-Leu |
| R² = 0.905, | -0.610 x D-Ser + 0.392 x L-Arg - 0.051 x L-Met - 0.183 x L-Ile + 0.264 x L-Tyr |
| R² = 0.904, | -0.520 x D-Ser - 0.181 x L-Asn + 0.333 x L-Arg + 0.088 x L-Lys - 0.340 x L-CysCys |
| R² = 0.904, | -0.572 x D-Ser - 0.197 x L-Gln + 0.367 x L-Arg - 0.172 x L-Ile + 0.247 x L-Tyr |
| R² = 0.904, | -0.520 x D-Ser + 0.334 x L-Arg - 0.235 x L-Asp + 0.276 x L-Val - 0.156 x L-Ile |
| R² = 0.904, | -0.417 x D-Ser + 0.267 x L-Arg - 0.250 x L-Gly + 0.221 x L-Val - 0.272 x L-CysCys |
| R² = 0.903, | -0.627 x D-Ser - 0.217 x L-Gln + 0.403 x L-Arg - 0.188 x L-Ile - 0.098 x L-Phe |
| R² = 0.903, | -0.568 x D-Ser + 0.365 x L-Arg - 0.104 x L-Pro + 0.302 x L-Val - 0.170 x L-Ile |
| R² = 0.902, | -0.565 x D-Ser + 0.116 x L-Ser + 0.363 x L-Arg + 0.300 x L-Val - 0.169 x L-Ile |
| R² = 0.902, | -0.656 x D-Ser - 0.227 x L-Gln + 0.421 x L-Arg - 0.120 x L-Pro - 0.054 x L-Met |
| R² = 0.902, | -0.463 x D-Ser + 0.297 x L-Arg - 0.278 x L-Gly + 0.078 x L-Lys - 0.303 x L-CysCys |
| R² = 0.902, | -0.697 x D-Ser + 0.447 x L-Arg - 0.127 x L-Pro - 0.058 x L-Met + 0.118 x L-Lys |
| R² = 0.902, | -0.531 x D-Ser + 0.341 x L-Arg + 0.282 x L-Val - 0.159 x L-Ile + 0.212 x L-Leu |
| R² = 0.902, | -0.466 x D-Ser + 0.299 x L-Arg + 0.248 x L-Val - 0.140 x L-Ile - 0.305 x L-CysCys |
| R² = 0.902, | -0.610 x D-Ser + 0.392 x L-Arg - 0.183 x L-Ile + 0.244 x L-Leu + 0.103 x L-Lys |
| R² = 0.901, | -0.462 x D-Ser - 0.310 x D-Ala + 0.296 x L-Arg + 0.245 x L-Val - 0.138 x L-Ile |
| R² = 0.901, | -0.673 x D-Ser + 0.432 x L-Arg + 0.024 x L-Thr - 0.202 x L-Ile + 0.114 x L-Lys |
| R² = 0.901, | -0.577 x D-Ser + 0.371 x L-Arg + 0.012 x L-Ala + 0.307 x L-Val - 0.173 x L-Ile |

Correlation
coefficient

# FIG. 9-2

| | Formula |
|---|---|
| $R^2 = 0.9,$ | -0.656 x D-Ser - 0.229 x L-Asn + 0.421 x L-Arg - 0.054 x L-Met + 0.111 x L-Lys |
| $R^2 = 0.9,$ | -0.624 x D-Ser - 0.218 x L-Asn + 0.401 x L-Arg - 0.187 x L-Ile - 0.098 x L-Phe |
| $R^2 = 0.9,$ | -0.655 x D-Ser + 0.134 x L-Ser + 0.420 x L-Arg - 0.196 x L-Ile + 0.111 x L-Lys |
| $R^2 = 0.9,$ | -0.681 x D-Ser + 0.437 x L-Arg - 0.206 x L-Glu + 0.014 x L-Ala - 0.057 x L-Met |
| $R^2 = 0.9,$ | -0.669 x D-Ser + 0.429 x L-Arg - 0.202 x L-Glu - 0.056 x L-Met + 0.113 x L-Lys |
| $R^2 = 0.9,$ | -0.593 x D-Ser + 0.381 x L-Arg - 0.108 x L-Pro - 0.049 x L-Met + 0.315 x L-Val |
| $R^2 = 0.9,$ | -0.697 x D-Ser + 0.447 x L-Arg - 0.127 x L-Pro - 0.058 x L-Met - 0.109 x L-Phe |
| $R^2 = 0.94,$ | -0.617 x D-Ser - 0.213 x L-Gln + 0.396 x L-Arg - 0.187 x L-Glu - 0.185 x L-Ile + 0.104 x L-Lys |
| $R^2 = 0.938,$ | -0.446 x D-Ser - 0.402 x D-Pro + 0.286 x L-Arg - 0.135 x L-Glu + 0.009 x L-Ala - 0.134 x L-Ile |
| $R^2 = 0.937,$ | -0.669 x D-Ser + 0.429 x L-Arg - 0.202 x L-Glu + 0.013 x L-Ala - 0.056 x L-Met - 0.201 x L-Ile |
| $R^2 = 0.936,$ | -0.540 x D-Ser + 0.346 x L-Arg - 0.244 x L-Asp - 0.163 x L-Glu - 0.162 x L-Ile + 0.233 x L-Tyr |
| $R^2 = 0.934,$ | -0.589 x D-Ser - 0.206 x L-Asn + 0.378 x L-Arg - 0.177 x L-Ile + 0.236 x L-Leu + 0.100 x L-Lys |
| $R^2 = 0.934,$ | -0.535 x D-Ser + 0.109 x L-Ser + 0.343 x L-Arg - 0.322 x L-Gly - 0.162 x L-Glu - 0.160 x L-Ile |
| $R^2 = 0.933,$ | -0.608 x D-Ser + 0.124 x L-Ser - 0.210 x L-Gln + 0.390 x L-Arg - 0.184 x L-Glu - 0.182 x L-Ile |
| $R^2 = 0.933,$ | -0.590 x D-Ser - 0.204 x L-Gln + 0.378 x L-Arg - 0.177 x L-Ile + 0.236 x L-Leu + 0.100 x L-Lys |
| $R^2 = 0.933,$ | -0.653 x D-Ser + 0.419 x L-Arg - 0.198 x L-Glu - 0.119 x L-Pro - 0.054 x L-Met - 0.196 x L-Ile |
| $R^2 = 0.932,$ | -0.545 x D-Ser + 0.350 x L-Arg - 0.328 x L-Gly - 0.165 x L-Glu + 0.011 x L-Ala - 0.164 x L-Ile |
| $R^2 = 0.932,$ | -0.597 x D-Ser + 0.383 x L-Arg - 0.181 x L-Glu - 0.050 x L-Met - 0.179 x L-Ile + 0.258 x L-Tyr |
| $R^2 = 0.931,$ | -0.507 x D-Ser - 0.340 x D-Ala - 0.175 x L-Gln + 0.326 x L-Arg - 0.152 x L-Ile + 0.086 x L-Lys |
| $R^2 = 0.931,$ | -0.519 x D-Ser + 0.333 x L-Arg - 0.312 x L-Gly - 0.156 x L-Ile + 0.208 x L-Leu + 0.088 x L-Lys |
| $R^2 = 0.931,$ | -0.516 x D-Ser + 0.331 x L-Arg - 0.156 x L-Glu + 0.274 x L-Val - 0.155 x L-Ile + 0.223 x L-Tyr |
| $R^2 = 0.93,$ | -0.396 x D-Ser - 0.357 x D-Pro + 0.254 x L-Arg - 0.120 x L-Glu + 0.210 x L-Val - 0.119 x L-Ile |
| $R^2 = 0.93,$ | -0.587 x D-Ser + 0.017 x L-His + 0.377 x L-Arg + 0.312 x L-Val - 0.176 x L-Ile + 0.099 x L-Lys |
| $R^2 = 0.93,$ | -0.584 x D-Ser + 0.375 x L-Arg - 0.264 x L-Asp - 0.177 x L-Glu - 0.175 x L-Ile - 0.092 x L-Phe |
| $R^2 = 0.93,$ | -0.526 x D-Ser + 0.337 x L-Arg - 0.316 x L-Gly + 0.010 x L-Ala - 0.158 x L-Ile + 0.210 x L-Leu |
| $R^2 = 0.93,$ | -0.488 x D-Ser + 0.313 x L-Arg - 0.293 x L-Gly - 0.089 x L-Pro + 0.259 x L-Val - 0.146 x L-Ile |
| $R^2 = 0.93,$ | -0.481 x D-Ser + 0.309 x L-Arg - 0.289 x L-Gly - 0.144 x L-Ile + 0.193 x L-Leu + 0.208 x L-Tyr |
| $R^2 = 0.93,$ | -0.604 x D-Ser + 0.388 x L-Arg - 0.183 x L-Glu - 0.050 x L-Met - 0.181 x L-Ile + 0.242 x L-Leu |
| $R^2 = 0.93,$ | -0.653 x D-Ser + 0.419 x L-Arg - 0.198 x L-Glu - 0.054 x L-Met - 0.196 x L-Ile + 0.110 x L-Lys |
| $R^2 = 0.93,$ | -0.548 x D-Ser + 0.352 x L-Arg - 0.166 x L-Glu - 0.164 x L-Ile + 0.219 x L-Leu + 0.237 x L-Tyr |
| $R^2 = 0.93,$ | -0.587 x D-Ser + 0.376 x L-Arg + 0.021 x L-Thr + 0.312 x L-Val - 0.176 x L-Ile + 0.099 x L-Lys |
| $R^2 = 0.929,$ | -0.549 x D-Ser - 0.192 x L-Asn + 0.353 x L-Arg + 0.292 x L-Val - 0.165 x L-Ile + 0.093 x L-Lys |
| $R^2 = 0.929,$ | -0.532 x D-Ser - 0.184 x L-Gln + 0.342 x L-Arg - 0.161 x L-Glu + 0.283 x L-Val - 0.160 x L-Ile |
| $R^2 = 0.929,$ | -0.582 x D-Ser + 0.374 x L-Arg - 0.263 x L-Asp - 0.176 x L-Glu - 0.175 x L-Ile + 0.098 x L-Lys |
| $R^2 = 0.929,$ | -0.561 x D-Ser + 0.360 x L-Arg - 0.338 x L-Gly + 0.011 x L-Ala - 0.168 x L-Ile + 0.095 x L-Lys |
| $R^2 = 0.929,$ | -0.517 x D-Ser + 0.332 x L-Arg - 0.311 x L-Gly - 0.094 x L-Pro - 0.155 x L-Ile + 0.207 x L-Leu |
| $R^2 = 0.929,$ | -0.456 x D-Ser + 0.292 x L-Arg - 0.274 x L-Gly + 0.242 x L-Val - 0.137 x L-Ile + 0.197 x L-Tyr |
| $R^2 = 0.929,$ | -0.566 x D-Ser + 0.363 x L-Arg - 0.171 x L-Glu + 0.011 x L-Ala + 0.301 x L-Val - 0.170 x L-Ile |
| $R^2 = 0.928,$ | -0.408 x D-Ser - 0.368 x D-Pro + 0.012 x L-His + 0.262 x L-Arg + 0.217 x L-Val - 0.123 x L-Ile |
| $R^2 = 0.928,$ | -0.359 x D-Ser - 0.323 x D-Pro + 0.230 x L-Arg - 0.216 x L-Gly + 0.190 x L-Val - 0.108 x L-Ile |
| $R^2 = 0.928,$ | -0.436 x D-Ser - 0.393 x D-Pro + 0.280 x L-Arg - 0.132 x L-Glu - 0.131 x L-Ile + 0.074 x L-Lys |
| $R^2 = 0.928,$ | -0.573 x D-Ser - 0.198 x L-Gln + 0.368 x L-Arg - 0.259 x L-Asp - 0.172 x L-Ile + 0.097 x L-Lys |
| $R^2 = 0.928,$ | -0.474 x D-Ser + 0.304 x L-Arg - 0.285 x L-Gly - 0.144 x L-Glu + 0.252 x L-Val - 0.142 x L-Ile |
| $R^2 = 0.928,$ | -0.559 x D-Ser + 0.359 x L-Arg - 0.336 x L-Gly - 0.046 x L-Met - 0.168 x L-Ile + 0.094 x L-Lys |
| $R^2 = 0.928,$ | -0.488 x D-Ser + 0.313 x L-Arg - 0.293 x L-Gly + 0.259 x L-Val - 0.146 x L-Ile + 0.082 x L-Lys |
| $R^2 = 0.928,$ | -0.554 x D-Ser + 0.356 x L-Arg - 0.333 x L-Gly - 0.166 x L-Ile - 0.087 x L-Phe + 0.094 x L-Lys |
| $R^2 = 0.927,$ | -0.408 x D-Ser - 0.368 x D-Pro + 0.262 x L-Arg + 0.015 x L-Thr + 0.217 x L-Val - 0.122 x L-Ile |
| $R^2 = 0.927,$ | -0.642 x D-Ser + 0.019 x L-His - 0.222 x L-Gln + 0.412 x L-Arg - 0.193 x L-Ile + 0.108 x L-Lys |
| $R^2 = 0.927,$ | -0.485 x D-Ser + 0.099 x L-Ser + 0.311 x L-Arg - 0.292 x L-Gly + 0.258 x L-Val - 0.145 x L-Ile |
| $R^2 = 0.927,$ | -0.646 x D-Ser + 0.132 x L-Ser + 0.414 x L-Arg - 0.195 x L-Glu - 0.054 x L-Met - 0.194 x L-Ile |
| $R^2 = 0.927,$ | -0.619 x D-Ser - 0.214 x L-Gln + 0.397 x L-Arg - 0.187 x L-Glu + 0.012 x L-Ala - 0.186 x L-Ile |
| $R^2 = 0.927,$ | -0.566 x D-Ser - 0.195 x L-Gln + 0.363 x L-Arg - 0.171 x L-Glu - 0.170 x L-Ile + 0.227 x L-Leu |
| $R^2 = 0.927,$ | -0.643 x D-Ser - 0.222 x L-Gln + 0.413 x L-Arg + 0.013 x L-Ala - 0.193 x L-Ile + 0.109 x L-Lys |
| $R^2 = 0.927,$ | -0.633 x D-Ser - 0.219 x L-Gln + 0.406 x L-Arg - 0.190 x L-Ile - 0.099 x L-Phe + 0.107 x L-Lys |
| $R^2 = 0.927,$ | -0.510 x D-Ser + 0.327 x L-Arg - 0.230 x L-Asp - 0.154 x L-Glu + 0.271 x L-Val - 0.153 x L-Ile |
| $R^2 = 0.927,$ | -0.495 x D-Ser + 0.318 x L-Arg - 0.298 x L-Gly - 0.150 x L-Glu - 0.148 x L-Ile + 0.214 x L-Tyr |
| $R^2 = 0.927,$ | -0.561 x D-Ser + 0.360 x L-Arg - 0.337 x L-Gly + 0.011 x L-Ala - 0.168 x L-Ile - 0.088 x L-Phe |
| $R^2 = 0.926,$ | -0.406 x D-Ser - 0.366 x D-Pro + 0.260 x L-Arg - 0.183 x L-Asp - 0.123 x L-Glu - 0.122 x L-Ile |
| $R^2 = 0.926,$ | -0.408 x D-Ser - 0.368 x D-Pro + 0.262 x L-Arg + 0.008 x L-Ala + 0.217 x L-Val - 0.122 x L-Ile |
| $R^2 = 0.926,$ | -0.580 x D-Ser - 0.202 x L-Asn + 0.119 x L-Ser + 0.372 x L-Arg - 0.174 x L-Ile + 0.232 x L-Leu |

Correlation
coefficient

# FIG. 9-3

| | Formula |
|---|---|
| $R^2 = 0.926,$ | -0.589 x D-Ser + 0.378 x L-Arg - 0.266 x L-Asp - 0.178 x L-Glu + 0.012 x L-Ala - 0.177 x L-Ile |
| $R^2 = 0.926,$ | -0.578 x D-Ser + 0.371 x L-Arg - 0.261 x L-Asp - 0.175 x L-Glu - 0.106 x L-Pro - 0.173 x L-Ile |
| $R^2 = 0.926,$ | -0.476 x D-Ser + 0.306 x L-Arg - 0.215 x L-Asp - 0.144 x L-Glu - 0.143 x L-Ile - 0.311 x L-CysCys |
| $R^2 = 0.926,$ | -0.534 x D-Ser + 0.343 x L-Arg - 0.321 x L-Gly - 0.162 x L-Glu - 0.160 x L-Ile + 0.090 x L-Lys |
| $R^2 = 0.926,$ | -0.564 x D-Ser + 0.362 x L-Arg - 0.171 x L-Glu + 0.020 x L-Thr + 0.300 x L-Val - 0.169 x L-Ile |
| $R^2 = 0.926,$ | -0.604 x D-Ser + 0.388 x L-Arg - 0.183 x L-Glu + 0.012 x L-Ala - 0.181 x L-Ile + 0.242 x L-Leu |
| $R^2 = 0.926,$ | -0.563 x D-Ser + 0.361 x L-Arg - 0.170 x L-Glu - 0.047 x L-Met + 0.299 x L-Val - 0.169 x L-Ile |
| $R^2 = 0.926,$ | -0.652 x D-Ser + 0.418 x L-Arg - 0.197 x L-Glu - 0.054 x L-Met - 0.196 x L-Ile - 0.102 x L-Phe |
| $R^2 = 0.926,$ | -0.460 x D-Ser + 0.295 x L-Arg - 0.139 x L-Glu + 0.244 x L-Val - 0.138 x L-Ile - 0.301 x L-CysCys |
| $R^2 = 0.925,$ | -0.389 x D-Ser - 0.351 x D-Pro - 0.134 x L-Gln + 0.250 x L-Arg + 0.207 x L-Val - 0.117 x L-Ile |
| $R^2 = 0.925,$ | -0.652 x D-Ser + 0.019 x L-His + 0.419 x L-Arg - 0.197 x L-Glu - 0.196 x L-Ile + 0.110 x L-Lys |
| $R^2 = 0.925,$ | -0.543 x D-Ser - 0.190 x L-Asn + 0.111 x L-Ser + 0.349 x L-Arg + 0.288 x L-Val - 0.163 x L-Ile |
| $R^2 = 0.925,$ | -0.614 x D-Ser - 0.214 x L-Asn + 0.394 x L-Arg - 0.186 x L-Glu - 0.051 x L-Met - 0.184 x L-Ile |
| $R^2 = 0.925,$ | -0.575 x D-Ser + 0.118 x L-Ser + 0.369 x L-Arg - 0.260 x L-Asp - 0.174 x L-Glu - 0.173 x L-Ile |
| $R^2 = 0.925,$ | -0.526 x D-Ser - 0.182 x L-Gln + 0.337 x L-Arg - 0.316 x L-Gly - 0.158 x L-Ile + 0.089 x L-Lys |
| $R^2 = 0.925,$ | -0.641 x D-Ser - 0.221 x L-Gln + 0.411 x L-Arg + 0.023 x L-Thr - 0.192 x L-Ile + 0.108 x L-Lys |
| $R^2 = 0.925,$ | -0.539 x D-Ser + 0.346 x L-Arg - 0.324 x L-Gly - 0.163 x L-Glu - 0.045 x L-Met - 0.162 x L-Ile |
| $R^2 = 0.925,$ | -0.652 x D-Ser + 0.418 x L-Arg - 0.197 x L-Glu + 0.023 x L-Thr - 0.196 x L-Ile + 0.110 x L-Lys |
| $R^2 = 0.925,$ | -0.522 x D-Ser + 0.335 x L-Arg - 0.158 x L-Glu - 0.043 x L-Met - 0.157 x L-Ile - 0.341 x L-CysCys |
| $R^2 = 0.925,$ | -0.556 x D-Ser + 0.357 x L-Arg - 0.168 x L-Glu + 0.295 x L-Val - 0.167 x L-Ile + 0.094 x L-Lys |
| $R^2 = 0.925,$ | -0.594 x D-Ser + 0.381 x L-Arg - 0.180 x L-Glu - 0.178 x L-Ile + 0.238 x L-Leu + 0.100 x L-Lys |
| $R^2 = 0.925,$ | -0.593 x D-Ser + 0.380 x L-Arg + 0.021 x L-Thr + 0.012 x L-Ala + 0.315 x L-Val - 0.178 x L-Ile |
| $R^2 = 0.925,$ | -0.536 x D-Ser + 0.344 x L-Arg + 0.019 x L-Thr + 0.285 x L-Val - 0.161 x L-Ile + 0.232 x L-Tyr |
| $R^2 = 0.925,$ | -0.570 x D-Ser + 0.366 x L-Arg - 0.047 x L-Met - 0.171 x L-Ile + 0.228 x L-Leu + 0.246 x L-Tyr |
| $R^2 = 0.924,$ | -0.429 x D-Ser - 0.387 x D-Pro - 0.148 x L-Gln + 0.275 x L-Arg - 0.129 x L-Ile + 0.072 x L-Lys |
| $R^2 = 0.924,$ | -0.380 x D-Ser - 0.342 x D-Pro + 0.244 x L-Arg + 0.202 x L-Val - 0.114 x L-Ile + 0.164 x L-Tyr |
| $R^2 = 0.924,$ | -0.525 x D-Ser - 0.183 x L-Asn + 0.337 x L-Arg - 0.315 x L-Gly - 0.157 x L-Ile + 0.089 x L-Lys |
| $R^2 = 0.924,$ | -0.536 x D-Ser - 0.187 x L-Asn + 0.344 x L-Arg - 0.161 x L-Ile + 0.215 x L-Leu + 0.232 x L-Tyr |
| $R^2 = 0.924,$ | -0.511 x D-Ser + 0.105 x L-Ser + 0.328 x L-Arg - 0.307 x L-Gly - 0.153 x L-Ile + 0.205 x L-Leu |
| $R^2 = 0.924,$ | -0.638 x D-Ser - 0.220 x L-Gln + 0.409 x L-Arg - 0.053 x L-Met - 0.191 x L-Ile + 0.108 x L-Lys |
| $R^2 = 0.924,$ | -0.547 x D-Ser - 0.189 x L-Gln + 0.351 x L-Arg + 0.291 x L-Val - 0.164 x L-Ile + 0.092 x L-Lys |
| $R^2 = 0.923,$ | -0.433 x D-Ser - 0.390 x D-Pro + 0.278 x L-Arg - 0.131 x L-Glu - 0.079 x L-Pro - 0.130 x L-Ile |
| $R^2 = 0.923,$ | -0.434 x D-Ser - 0.391 x D-Pro + 0.279 x L-Arg - 0.131 x L-Glu - 0.130 x L-Ile - 0.068 x L-Phe |
| $R^2 = 0.923,$ | -0.402 x D-Ser - 0.363 x D-Pro + 0.258 x L-Arg + 0.214 x L-Val - 0.121 x L-Ile + 0.068 x L-Lys |
| $R^2 = 0.923,$ | -0.624 x D-Ser + 0.019 x L-His + 0.400 x L-Arg - 0.187 x L-Ile + 0.250 x L-Leu + 0.105 x L-Lys |
| $R^2 = 0.923,$ | -0.614 x D-Ser - 0.212 x L-Gln + 0.394 x L-Arg - 0.186 x L-Glu - 0.051 x L-Met - 0.184 x L-Ile |
| $R^2 = 0.923,$ | -0.539 x D-Ser + 0.346 x L-Arg - 0.244 x L-Asp - 0.163 x L-Glu - 0.162 x L-Ile + 0.216 x L-Leu |
| $R^2 = 0.923,$ | -0.492 x D-Ser + 0.316 x L-Arg - 0.296 x L-Gly + 0.010 x L-Ala + 0.261 x L-Val - 0.148 x L-Ile |
| $R^2 = 0.923,$ | -0.549 x D-Ser + 0.353 x L-Arg - 0.330 x L-Gly - 0.100 x L-Pro - 0.165 x L-Ile - 0.086 x L-Phe |
| $R^2 = 0.923,$ | -0.662 x D-Ser + 0.425 x L-Arg - 0.200 x L-Glu + 0.024 x L-Thr + 0.013 x L-Ala - 0.198 x L-Ile |
| $R^2 = 0.923,$ | -0.652 x D-Ser + 0.419 x L-Arg - 0.197 x L-Glu + 0.013 x L-Ala - 0.196 x L-Ile - 0.102 x L-Phe |
| $R^2 = 0.923,$ | -0.686 x D-Ser + 0.440 x L-Arg + 0.014 x L-Ala - 0.057 x L-Met - 0.206 x L-Ile + 0.116 x L-Lys |
| $R^2 = 0.923,$ | -0.486 x D-Pro + 0.346 x L-Arg - 0.324 x L-Gly + 0.011 x L-Ala + 0.286 x L-Val - 0.162 x L-Ile |
| $R^2 = 0.922,$ | -0.502 x D-Ser - 0.337 x D-Ala - 0.175 x L-Asn + 0.322 x L-Arg - 0.151 x L-Ile + 0.085 x L-Lys |
| $R^2 = 0.922,$ | -0.411 x D-Ser - 0.370 x D-Pro + 0.264 x L-Arg - 0.124 x L-Glu - 0.123 x L-Ile + 0.164 x L-Leu |
| $R^2 = 0.922,$ | -0.372 x D-Ser - 0.336 x D-Pro + 0.239 x L-Arg - 0.113 x L-Glu - 0.112 x L-Ile - 0.243 x L-CysCys |
| $R^2 = 0.922,$ | -0.659 x D-Ser + 0.020 x L-His + 0.423 x L-Arg - 0.199 x L-Glu - 0.055 x L-Met - 0.198 x L-Ile |
| $R^2 = 0.922,$ | -0.562 x D-Ser + 0.017 x L-His + 0.361 x L-Arg - 0.170 x L-Glu + 0.299 x L-Val - 0.169 x L-Ile |
| $R^2 = 0.922,$ | -0.605 x D-Ser - 0.211 x L-Asn + 0.388 x L-Arg - 0.183 x L-Glu - 0.181 x L-Ile + 0.102 x L-Lys |
| $R^2 = 0.922,$ | -0.591 x D-Ser - 0.206 x L-Asn + 0.379 x L-Arg + 0.012 x L-Ala - 0.177 x L-Ile + 0.236 x L-Leu |
| $R^2 = 0.922,$ | -0.504 x D-Ser - 0.176 x L-Asn + 0.324 x L-Arg + 0.268 x L-Val - 0.151 x L-Ile + 0.218 x L-Tyr |
| $R^2 = 0.922,$ | -0.542 x D-Ser + 0.111 x L-Ser - 0.187 x L-Gln + 0.348 x L-Arg + 0.288 x L-Val - 0.163 x L-Ile |
| $R^2 = 0.922,$ | -0.545 x D-Ser + 0.112 x L-Ser + 0.350 x L-Arg - 0.328 x L-Gly - 0.164 x L-Ile + 0.092 x L-Lys |
| $R^2 = 0.922,$ | -0.587 x D-Ser + 0.120 x L-Ser + 0.377 x L-Arg - 0.178 x L-Glu - 0.176 x L-Ile + 0.235 x L-Leu |
| $R^2 = 0.922,$ | -0.668 x D-Ser + 0.137 x L-Ser + 0.428 x L-Arg - 0.055 x L-Met - 0.200 x L-Ile + 0.113 x L-Lys |
| $R^2 = 0.922,$ | -0.555 x D-Ser - 0.192 x L-Gln + 0.356 x L-Arg - 0.168 x L-Glu - 0.167 x L-Ile + 0.240 x L-Tyr |
| $R^2 = 0.922,$ | -0.615 x D-Ser + 0.395 x L-Arg - 0.278 x L-Asp + 0.012 x L-Ala - 0.051 x L-Met - 0.185 x L-Ile |
| $R^2 = 0.922,$ | -0.486 x D-Ser + 0.312 x L-Arg - 0.292 x L-Gly + 0.258 x L-Val - 0.146 x L-Ile - 0.076 x L-Phe |
| $R^2 = 0.922,$ | -0.553 x D-Ser + 0.355 x L-Arg - 0.167 x L-Glu - 0.101 x L-Pro + 0.293 x L-Val - 0.166 x L-Ile |
| $R^2 = 0.922,$ | -0.581 x D-Ser + 0.373 x L-Arg - 0.176 x L-Glu - 0.106 x L-Pro - 0.174 x L-Ile + 0.251 x L-Tyr |
| $R^2 = 0.922,$ | -0.518 x D-Ser + 0.333 x L-Arg - 0.157 x L-Glu + 0.275 x L-Val - 0.155 x L-Ile + 0.207 x L-Leu |
| $R^2 = 0.922,$ | -0.533 x D-Ser + 0.342 x L-Arg - 0.044 x L-Met + 0.283 x L-Val - 0.160 x L-Ile + 0.230 x L-Tyr |

34

# FIG. 10

| ID | D-Ser | D-Ala | D-Pro | D-Asn | Inulin clearance_1.73 |
|---|---|---|---|---|---|
| | (nmol/mL) | (nmol/mL) | (nmol/mL) | (nmol/mL) | (mL/min/1.73) |
| #1 | 1.86 | 0.76 | 0.67 | 0.17 | 68.58 |
| #2 | 2.02 | 0.99 | 0.43 | 0.16 | 93.81 |
| #3 | 2.31 | 1.81 | 1.09 | 0.20 | 94.24 |
| #4 | 1.56 | 1.83 | 0.47 | 0.14 | 98.12 |
| #5 | 1.39 | 1.03 | 0.86 | 0.18 | 95.58 |
| #6 | 1.88 | 1.75 | 0.52 | 0.13 | 118.91 |
| #7 | 1.85 | 0.44 | 0.16 | 0.07 | 119.02 |
| #8 | 1.58 | 0.40 | 0.48 | 0.12 | 82.28 |
| #9 | 1.38 | 0.42 | 0.31 | 0.12 | 98.81 |
| #10 | 1.99 | 3.98 | 0.57 | 0.18 | 94.31 |
| #11 | 0.93 | 0.81 | 1.00 | 0.16 | 114.15 |
| #12 | 1.15 | 0.67 | 0.48 | 0.15 | 94.00 |
| #13 | 1.13 | 11.49 | 1.00 | 0.16 | 117.72 |
| #14 | 1.15 | 0.86 | 0.73 | 0.14 | 100.35 |
| #15 | 0.96 | 1.95 | 0.36 | 0.13 | 96.96 |
| #21 | 9.42 | 13.02 | 2.88 | 1.08 | 7.79 |
| #22 | 4.08 | 1.28 | 0.78 | 0.33 | 46.04 |
| #23 | 2.99 | 1.56 | 0.64 | 0.35 | 24.49 |
| #24 | 1.80 | 2.05 | 0.92 | 0.20 | 58.90 |
| #25 | 2.39 | 0.85 | 0.85 | 0.18 | 74.86 |
| #26 | 1.68 | 1.08 | 0.52 | 0.18 | 52.99 |
| #27 | 0.97 | 0.54 | 0.47 | 0.10 | 88.58 |
| #28 | 1.41 | 0.62 | 0.45 | 0.11 | 41.04 |
| #29 | 1.25 | 0.52 | 0.44 | 0.14 | 99.02 |
| #30 | 8.22 | 3.67 | 2.22 | 1.29 | 15.03 |
| #31 | 8.07 | 17.97 | 14.69 | 1.10 | 11.25 |

# FIG. 11

EP 4 083 628 B1

Correlation coefficient

| | Formula |
|---|---|
| $R^2 = 0.833,$ | $-0.4758 \times$ D-Asn $-0.4615 \times$ D-Ser |
| $R^2 = 0.822,$ | $-0.6406 \times$ D-Asn $-0.4166 \times$ D-Ala |
| $R^2 = 0.819,$ | $-0.5149 \times$ D-Asn $-0.4502 \times$ D-Pro |
| $R^2 = 0.769,$ | $-0.6065 \times$ D-Ser $-0.4066 \times$ D-Ala |
| $R^2 = 0.787,$ | $-0.4964 \times$ D-Ser $-0.4475 \times$ D-Pro |
| $R^2 = 0.647,$ | $-0.3931 \times$ D-Ala $-0.5286 \times$ D-Pro |
| $R^2 = 0.832,$ | $-0.3903 \times$ D-Asn $-0.3786 \times$ D-Ser $-0.2538 \times$ D-Ala |
| $R^2 = 0.834,$ | $-0.3419 \times$ D-Asn $-0.3316 \times$ D-Ser $-0.299 \times$ D-Pro |
| $R^2 = 0.828,$ | $-0.4196 \times$ D-Asn $-0.2729 \times$ D-Ala $-0.3669 \times$ D-Pro |
| $R^2 = 0.784,$ | $-0.3961 \times$ D-Ser $-0.2656 \times$ D-Ala $-0.357 \times$ D-Pro |
| $R^2 = 0.83,$ | $-0.2942 \times$ D-Asn $-0.2854 \times$ D-Ser $-0.1913 \times$ D-Ala $-0.2573 \times$ D-Pro |

Correlation coefficient

| | Formula |
|---|---|
| $R^2 = 0.705,$ | $-60.6925 \times$ D-Asn $-12.1334 \times$ D-Ser $+15.2991 \times$ D-Asn$^2$ $+5.4724 \times$ D-Ser$^2$ $+57.2133$ |
| $R^2 = 0.754,$ | $-84.6481 \times$ D-Asn $+21.8252 \times$ D-Ala $+22.2784 \times$ D-Asn$^2$ $-4.5483 \times$ D-Ala$^2$ $+60.1379$ |
| $R^2 = 0.746,$ | $-101.1348 \times$ D-Asn $+88.916 \times$ D-Pro $+24.0407 \times$ D-Asn$^2$ $-16.6259 \times$ D-Pro$^2$ $+70.0565$ |
| $R^2 = 0.668,$ | $-42.8827 \times$ D-Ser $+14.2722 \times$ D-Ala $+5.9368 \times$ D-Ser$^2$ $-3.871 \times$ D-Ala$^2$ $+75.1997$ |
| $R^2 = 0.65,$ | $-43.7637 \times$ D-Ser $+7.8785 \times$ D-Pro $+7.3129 \times$ D-Ser$^2$ $-1.5406 \times$ D-Pro$^2$ $+71.6358$ |
| $R^2 = 0.496,$ | $14.2071 \times$ D-Ala $-125.4857 \times$ D-Pro $-0.2983 \times$ D-Ala$^2$ $+22.2179 \times$ D-Pro$^2$ $+56.1095$ |
| $R^2 = 0.754,$ | $-79.1634 \times$ D-Asn $-4.0209 \times$ D-Ser $+21.6913 \times$ D-Ala $+21.4614 \times$ D-Asn$^2$ $+0.1396 \times$ D-Ser$^2$ $-4.5052 \times$ D-Ala$^2$ $+60.7477$ |
| $R^2 = 0.746,$ | $-101.9542 \times$ D-Asn $+0.8193 \times$ D-Ser $+92.364 \times$ D-Pro $+24.5182 \times$ D-Asn$^2$ $-0.856 \times$ D-Ser$^2$ $-17.3025 \times$ D-Pro$^2$ $+71.0711$ |
| $R^2 = 0.777,$ | $-103.618 \times$ D-Asn $+25.4238 \times$ D-Ala $+69.9552 \times$ D-Pro $+24.7593 \times$ D-Asn$^2$ $-10.2734 \times$ D-Ala$^2$ $-11.2492 \times$ D-Pro$^2$ $+74.0739$ |
| $R^2 = 0.671,$ | $-43.2245 \times$ D-Ser $+18.8918 \times$ D-Ala $-16.4252 \times$ D-Pro $+7.4924 \times$ D-Ser$^2$ $-6.5372 \times$ D-Ala$^2$ $+3.95 \times$ D-Pro$^2$ $+72.4694$ |
| $R^2 = 0.778,$ | $-111.8837 \times$ D-Asn $+4.1507 \times$ D-Ser $+28.2982 \times$ D-Ala $+71.675 \times$ D-Pro $+25.1118 \times$ D-Asn$^2$ $+1.5569 \times$ D-Ser$^2$ $-12.7494 \times$ D-Ala$^2$ $-10.7688 \times$ D-Pro$^2$ $+74.1567$ |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013140785 A
- US 2019317106 A1
- JP 4291628 B


### Non-patent literature cited in the description

- **FUKUSHIMA, T. et al.** *Biol. Pharm. Bull.*, 1995, vol. 18, 1130
- **NAGATA Y.** *Viva Origino*, vol. 18 (2), 1990
- **ISHIDA et al.** *Kitasato Medical Journal*, 1993, vol. 23, 51-62
- **YONG HUANG et al.** *Biol. Pharm. Bull.*, 1998, vol. 21 (2), 156-162
- **Y. NAGATA et al.** *Clinical Science*, 1987, vol. 73, 105
- *Analytical Biochemistry*, 1985, vol. 150, 238
- **A. D'ANIELLO et al.** *Comparative Biochemistry and Physiology Part B*, 1980, vol. 66, 319
- *Journal of Neurochemistry*, 1977, vol. 29, 1053
- **A. BERNEMAN et al.** *Journal of Microbial & Biochemical Technology*, 2010, vol. 2, 139
- **W. G. GUTHEIL et al.** *Analytical Biochemistry*, 2000, vol. 287, 196
- **G. MOLLA et al.** *Methods in Molecular Biology*, 2012, vol. 794, 273
- **T. ITO et al.** *Analytical Biochemistry*, 2007, vol. 371, 167
- **T. OHGUSU et al.** *Analytical Biochemistry*, 2006, vol. 357, 15
- **H. HASEGAWA et al.** *Journal of Mass Spectrometry*, 2011, vol. 46, 502
- **M. C. WALDHIER et al.** *Analytical and Bioanalytical Chemistry*, 2009, vol. 394, 695
- **A. HASHIMOTO ; T. NISHIKAWA et al.** *FEBS Letters*, 1992, vol. 296, 33
- **H. BRUCKNER ; A. SCHIEBER**. *Biomedical Chromatography*, 2001, vol. 15, 166
- **M. JUNGE et al.** *Chirality*, 2007, vol. 19, 228
- **M. C. WALDHIER et al.** *Journal of Chromatography A*, 2011, vol. 1218, 4537
- **H. MIAO et al.** *Analytical Chemistry*, 2005, vol. 77, 7190
- **D. L. KIRSCHNER et al.** *Analytical Chemistry*, 2007, vol. 79, 736
- **F. KITAGAWA ; K. OTSUKA**. *Journal of Chromatography B*, 2011, vol. 879, 3078
- **G. THORSEN ; J. BERGQUIST**. *Journal of Chromatography B*, 2000, vol. 745, 389
- **N. NIMURA ; T. KINOSHITA**. *Journal of Chromatography*, 1986, vol. 352, 169
- **A. HASHIMOTO et al.** *Journal of Chromatography*, 1992, vol. 582, 41
- **H. BRUCKNER et al.** *Journal of Chromatography A*, 1994, vol. 666, 259
- **N. NIMURA et al.** *Analytical Biochemistry*, 2003, vol. 315, 262
- **C. MULLER et al.** *Journal of Chromatography A*, 2014, vol. 1324, 109
- **S. EINARSSON et al.** *Analytical Chemistry*, 1987, vol. 59, 1191
- **E. OKUMA ; H. ABE**. *Journal of Chromatography B*, 1994, vol. 660, 243
- **Y. GOGAMI et al.** *Journal of Chromatography B*, 2011, vol. 879, 3259
- **Y. NAGATA et al.** *Journal of Chromatography*, 1992, vol. 575, 147
- **S. A. FUCHS et al.** *Clinical Chemistry*, 2008, vol. 54, 1443
- **D. GORDES et al.** *Amino Acids*, 2011, vol. 40, 553
- **D. JIN et al.** *Analytical Biochemistry*, 1999, vol. 269, 124
- **J. Z. MIN et al.** *Journal of Chromatography B*, 2011, vol. 879, 3220
- **T. SAKAMOTO et al.** *Analytical and Bioanalytical Chemistry*, 2016, vol. 408, 517
- **W. F. VISSER et al.** *Journal of Chromatography A*, 2011, vol. 1218, 7130
- **Y. XING et al.** *Analytical and Bioanalytical Chemistry*, 2016, vol. 408, 141
- **K. IMAI et al.** *Biomedical Chromatography*, 1995, vol. 9, 106
- **T. FUKUSHIMA et al.** *Biomedical Chromatography*, 1995, vol. 9, 10
- **R. J. REISCHL et al.** *Journal of Chromatography A*, 2011, vol. 1218, 8379
- **R. J. REISCHL ; W. LINDNER**. *Journal of Chromatography A*, 2012, vol. 1269, 262
- **S. KARAKAWA et al.** *Journal of Pharmaceutical and Biomedical Analysis*, 2015, vol. 115, 123

- **HAMASE, K** ; **ZAITSU, K**. *Bunseki Kagaku*, 2004, vol. 53, 677-690